# EUROPÄISCHE PATENTSCHRIFT

(11) **EP 1 414 783 B1**
(45) Veröffentlichungstag und Bekanntmachung des Hinweises auf die Patenterteilung: **15.07.2009**
(21) Anmeldenummer: 02767327.6
(22) Anmeldetag: 06.08.2002
(51) Int. Cl.: C07C 209/28, C07C 213/02, C07C 231/02, C07C 231/08, C07D 213/38, C07F 17/02

(54) **VERFAHREN ZUR HERSTELLUNG VON AMINEN DURCH REDUKTIVE AMINIERUNG VON CARBONYLVERBINDUNGEN UNTER TRANSFER-HYDRIERUNGSBEDINGUNGEN**
METHOD FOR THE PRODUCTION OF AMINES BY REDUCTIVE AMINATION OF CARBONYL COMPOUNDS UNDER TRANSFER-HYDROGENATION CONDITIONS
PROCEDE DE PRODUCTION D'AMINES PAR AMINATION DE COMPOSES CARBONYLE DANS DES CONDITIONS D'HYBRIDATION PAR TRANSFERT

(30) Priorität: 09.08.2001 DE 10138140
(43) Veröffentlichungstag der Anmeldung: 06.05.2004
(73) Patentinhaber: Evonik Degussa GmbH, 45128 Essen (DE)
(72) Erfinder: BÖRNER, Armin, 18055 Rostock (DE); DINGERDISSEN, Uwe, 64342 Seeheim (DE); KADYROV, Renat, 65931 Frankfurt (DE); RIERMEIER, Thomas, 65439 Flörsheim (DE); TARAROV, Vitali, Moskau, 113525 (RU)
(86) Internationale Anmeldenummer: PCT/EP2002/008748
(87) Internationale Veröffentlichungsnummer: WO 2003/014061

(56) Entgegenhaltungen:
- EP-A- 0 535 484
- US-A- 4 851 548
- CHEMICAL ABSTRACTS, vol. 53, no. 4, 25. Februar 1959 (1959-02-25) Columbus, Ohio, US; abstract no. 3112i, A.N. KOST: "Synthesis of amines by the method of Leuckart" Spalte 3112; XP002224161 in der Anmeldung erwähnt & NAUCH. DOKLADY VYSSHEI SHKOLY, KHIM. I KHIM. TEKHNOL., Nr. 1, 1958, Seiten 125-129,
- JOURNAL OF THE CHEMICAL SOCIETY, PERKIN TRANSACTIONS 2, 2000,
- DANIEL SIEGFRIED STRASSNER: "Synthese, in-vitro-Pharmakologie und Struktur-Wirkungsbeziehungen chiraler 5-HT"A-Rezeptorantagonisten der 3-Phenylchinolin-Reihe" 2003, DISSERTATION (FREIE UNIVERSITÄT BERLIN) * Seite 20 * * Seiten 24,25 *

## Beschreibung

Die Erfindung beschreibt die Herstellung von Aminen durch die Reaktion von Aldehyden oder Ketonen mit Ammoniak bzw. primären oder sekundären Aminen in Gegenwart eines Wasserstoff-Donors und in Anwesenheit von homogenen Metallkatalysatoren unter milden Bedingungen.

Racemische und enantiomerenreine Amine spielen eine dominierende Rolle in zahlreichen komplexen Naturstoffen wie beispielsweise den Alkaloiden, Vitaminen oder Aminosäuren, deren chemische, pharmazeutische und industrielle Bedeutung unbestritten ist. Als chemische Zwischenprodukte finden Amine u.a. Anwendung in der Synthese von Pharmazeutika, Agrochemikalien, Nahrungsmittelzusatzstoffen, Farbstoffen oder Kosmetika. Für den Bereich der Wirkstoffe spielen dabei Aminosäuren und Aminoalkohole eine überragende Rolle.

Für die Synthese von unfunktionalisierten und funktionalisierten Aminen spielt die reduktive Aminierung (Hydroaminierung) von Ketonen und Aldehyden eine große Rolle. Eine beträchtliche präparative und technische Bedeutung hat die katalytische Reduktion mit Wasserstoff (W.S. Emerson in Organic Reactions, Vol. 4, John Wiley & Sons, New York, 1948, pp. 174-255, Rylander Catalytic Hydrogenation over Platinum Metals, Academic Press, New York, 1967, S. 291-303; Catalytic Hydrogenation in Organic Synthesis, Academic Press, New York, 1979, 165 ff; M.V. Klyuev, M.L. Khidekel Ruiss. Chem. Rev. 1980, 49, 14-27; Rylander Hydrogenation Methods; Academic Press, New York, 1985, S. 82-93; V.A.Tarasevich, N.G. Kozlov Ruiss. Chem. Rev. 1999, 68, 55-72). Die Verwendung von Wasserstoff als Reduktionsmittel erfordert Hochdruckapparaturen, die hohe Betriebskosten verursachen und einen beträchtlichen technischen Aufwand beim Bau und Betrieb entsprechender Anlagen darstellen.

Reduktionen mit Metallhydriden wie Natrium Borhydrid (G. W. Gribble Chem. Soc. Rev. 1998, 27, 395-404), Natrium Cyanoborhydrid (R.O. Hutchins, N.R. Natale Org. Prep. Proceed. Int. 1979, 11, 201) oder Natrium Triacetoxyborhydrid (A.F. Abdel-Magid, C.A. Maryanoff in Reductions in Organic Synthesis, ACS Symp. Ser. Vol. 641, 1996, 201-216) verlaufen im allgemeinen unter milderen Bedingungen, aber sind mit beträchtlichen Problemen wie Explosionsgefahr und Giftigkeit verbunden.

Andere Reduktionsmittel wie Aluminium oder Zink (F. Möller, R. Schröter in Houben-Weyl, Methoden der Organischen Chemie, Bd. XI/1, Hrsg. E. Müller; Thieme Verlag, Stuttgart, 1957, 667-669), sowie die elektrolytische Reduktion (Yu.D. Smimov, L.A. Fedorova, A.P. Tomilov Elektrokhimia 1992, 28, 588-599) sind von geringerer Bedeutung. Die Herstellung von chiralen Aminen erfordert hier die Verwendung stöchiometrischer Mengen an oft schwer zugänglichen chiralen Hilfsverbindungen und in der Regel deren anschließende Abspaltung.

Sehr hohe optische Ausbeuten werden bei der enzymatische Transaminierung erreicht (R. O. Duthaler Tetrahedron. 1994, 50, 1539-1650). Die Methode ist allerdings hauptsächlich auf die Herstellung von Aminosäuren beschränkt. Die entsprechenden Enzyme sind darüber hinaus nur schwer zugänglich. Ferner erfordert die Abtrennung der wäßrigen Pufferlösungen einen hohen Aufwand.

Bei der reduktiven Aminierung nach Leuckart-Wallach (M.L.Moore in Organic Reactions, Vol. 5, John Wiley & Sons, New York, 1949, pp. 301-330) wird Ameisensäure als Reduktionsmittel eingesetzt. Im Labormaßstab hat sich die Verwendung der leicht handhabbaren organischen Wassersoff-Donoren als Reduktionsmittel bewährt, da diese nicht explosiv und kaum toxisch sind. Durch die Variation dieser Wasserstoff-Donoren lässt sich auch die Selektivität der Aminierungsreaktion beeinflussen. Die Leuckart-Wallach Reaktion wird durch Zusatz von trägergebundenen, heterogenen Hydrierungskatalysatoren wie Nickel oder Kobalt (A.N. Kost Nauch. Doklady Vysshei Shkoly, Khim. l Khim. Tekhnol. 1958, 125-129, C.A. **1959,** *53*, 3112i), Nickel-Aluminium-Legierungen oder Raneynickel (DRP 861844 (1943), Chem. Zentralblatt, 1953, 5926) beschleunigt.

Auch Lewis-Säuren wie Magnesiumchlorid, Zinkchlorid, Eisenchlorid (J. F. Bunnett, J. L. Marks J. Am. Chem. Soc. 1949, 71, 1587-1589) oder Aluminiumchlorid (US 4851548) katalysieren die Aminierung von Carbonylverbindungen. Die Umsetzung von Ketonen verläuft dabei nur bei hohen Temperaturen von 150 bis 200°C mit befriedigenden Ausbeuten. Mit den beschriebenen Katalysatoren ist jedoch keine enantioselektive Reaktionsführung durchführbar.

Phenylethylamine lassen sich durch die reduktive Aminierung der korrespondierenden ketone unter Verwendung von Ammoniumacetat und natriumcyanoborhydrid herstellen (k. kinbara, et al., J. Chem. Soc., perkin Trans 2., 2000, 1339 -1347).

Der Erfindung liegt daher die Aufgabe zugrunde, ein katalytisches Verfahren bereitzustellen, mit dem die Aminierung von Carbonylverbindungen unter milden Bedingungen ermöglicht wird und das bei Bedarf die Synthese von enantiomerenreinen oder enantiomerenangereicherten Aminen erlaubt.

Es wurde nun überraschenderweise gefunden, daß die gewünschten Amine sehr effizient durch reduktive Hydridotransfer-Aminierung von Ketonen und Aldehyden mit Ammoniak, primären oder sekundären Aminen in Gegenwart eines Wasserstoffdonors und von katalytisch wirkenden Übergangsmetallkomplexen enthaltend mindestens ein Metall aus der Gruppe Fe, Ru, Os, Co, Rh, Ir, Ni, Pd, Pt unter sehr milden Bedingungen zugänglich sind. Vorzugsweise werden homogene Metallatom-Ligand-Komplexe mit Zentralatomen aus der Gruppe Ru oder Rh verwendet.

Die milden Reaktionsbedingungen erlauben den Einsatz von Carbonylverbindungen unterschiedlichster Struktur als Edukt für die reduktive Hydridotransfer-Aminierung. Weiterhin wird durch die milden Bedingungen bei Verwendung von chiralen Liganden eine enantioselektive Reaktionsführung möglich. Die verwendeten Übergangsmetallkatalysatoren der achten Nebengruppe liefern in der reduktiven Aminierung gute bis sehr gute Ausbeuten an gewünschtem Amin. Gleichzeitig kann ein sehr hohes Amin/Alkohol Verhältnis in den Produkten realisiert werden.

Als Edukte können z. B. Carbonylverbindungen der Formel (I) 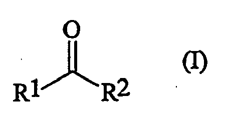 mit Ammoniak, primären oder sekundären Aminen der Formel (II) 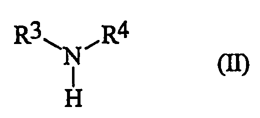 zu Verbindungen der allgemeinen Formel (III) umgesetzt werden, 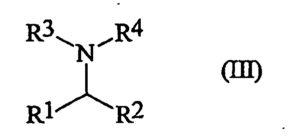 worin die Reste
- R¹ bis R⁴: unabhängig voneinander aus der Gruppe von Wasserstoff, (C₁-C₂₄)-Alkyl, (C₂-C₂₄)-Alkenyl, (C₂-C₂₄)-Alkinyl, (C₅-C₁₀)-Aryl, CF₃, CHO, CO-Alkyl-(C₁-C₈), CO-Aryl-(C₅-C₁₀), COO-Alkyl-(C₁-C₈), COO-Aryl-(C₅-C₁₀), C(=NAlkyl-(C₁-C₈))O-Alkyl-(C₁-C₈), C(=NAryl-(C₆-C₁₀))O-Alkyl-(C₁-C₈), C(=NAlkyl-(C₁-C₈))O-Aryl-(C₅-C₁₀), C(=NAryl-(C₅-C₁₀))O-Aryl-(C₅-C₁₀), CONH₂, CONHAlkyl-(C₁-C₈), CON(Alkyl-(C₁-C₈))₂, CONHAryl-(C₅-C₁₀), CON(Aryl-(C₅-C₁₀))₂, PO(Aryl-(C₅-C₁₀))₂, PO(Alkyl-(C₁-C₄))₂, PO(Alkyl-(C₁-C₄))(O-Alkyl-(C₁-C₆)), PO(Alkyl-(C₁-C₆))(OAryl-(C₅-C₁₀)), PO(Aryl-(C₅-C₁₀))(OAlkyl-(C₁-C₆)), PO(O-Alkyl-(C₁-C₆))₂, PO(OAryl-(C₅-C₁₀))₂, PO(O-Alkyl-(C₁-C₆))(O-Aryl-(C₅-C₁₀)), SO₂-OAlkyl-(C₁-C₄), SO₂-OAryl-(C₅-C₁₀), SO₂-Alkyl-(C₁-C₆), SO₂-Aryl-(C₅-C₁₀), SO-Alkyl-(C₁-C₈), SO-Aryl-(C₅-C₁₀) oder Si(Alkyl-(C₁-C₈))₃, Si(C₁-C₁₀-Alkyl/C₅-C₁₀-Aryl)₃, wobei M ein Kation z. B. ein Li⁺, Na⁺, K⁺, Mg²⁺, Ca²⁺, NH₄⁺, N(C₁-C₁₀-Alkyl)₄⁺, N(H/C₁-C₁₀-Alkyl/C₆-C₁₀-Aryl)₄⁺ darstellt, ausgewählt werden können und/oder
- R¹ bis R²: unabhängig voneinander aus der Gruppe CH(O-Alken-(C₂-C₈)-O), C(O-Alken-(C₂-C₈)-O)-Alkyl-(C₁-C₈), C(O-Alken-(C₂-C₈)-O)-Aryl-(C₅-C₁₀), CH(O-Alkyl-(C₁-C₈))₂, C(O-Alkyl-(C₁-C₈))₂-Alkyl-(C₁-C₈), C(O-Alkyl-(C₁-C₈))₂-Aryl-(C₅-C₁₀), CHO, CN, COOH, COOM, POH(Alkyl-(C₁-C₆)), POH(Aryl-(C₅-C₁₀)), PO₃H₂, PO₃M₂, SO₃H, SO₃M, wobei M ein Kation z. B. ein Li⁺, Na⁺, K⁺, Mg²⁺, Ca²⁺, NH₄⁺, N(C₁-C₁₀-Alkyl)₄⁺, N(H/C₁-C₁₀-Alkyl/C₆-C₁₀-Aryl)₄⁺ darstellt, ausgewählt werden können
und/oder
- R³ und R⁴: unabhängig voneinander aus der Gruppe O-Alkyl-(C₁-C₈), O-Aryl-(C₅-C₁₀), Fluor, OH, NH₂, NH(Alkyl-(C₁-C₈)), N(Alkyl-(C₁-C₈))₂, NH(Aryl-(C₅-C₁₀)), NHCO-Alkyl-(C₁-C₄), NHCO-Aryl-(C₅-C₁₀), NHCOO-Alkyl-(C₁-C₈), NHCOO-Aryl-(C₅-C₁₀) darstellt, ausgewählt werden können,
wobei Alkyl für einen nichtverzweigten oder verzweigten aliphatischen oder cyclischen Rest, Alkenyl für einen olefinischen Kohlenwasserstoff, Alkinyl für einen Acetylenkohlenwassersoff und Aryl für einen aromatischen Rest steht, wobei jeweils bis zu 4 Kohlenstoffatome durch Stickstoff-, Phosphor-, Silicium-, Schwefel- oder Sauerstoffatom ausgetauscht sein können.

Bevorzugt enthalten die heteroaliphatischen oder heteroaromatischen Gruppen der Edukte ein oder zwei Stickstoffatome oder ein Sauerstoff-, Schwefel- oder Phosphoratom.

Beispiele für besonders bevorzugte Alkylgruppen oder Heterocycloalkylgruppen sind Methyl-, Ethyl-, n-Propyl-, iso-Propyl-, n-Butyl-, iso-Butyl-, sec-Butyl-, t-Butyl-, Cylopentyl-, Cyclohexyl-, Tetrahydrofuryl-, Tetrahydropiryl-, Tetrahydrothiophenyl-, Piperidinyl-, Morpholinyl- und Phosphorinanylreste.
Beispiele für besonders bevorzugte Arylgruppen oder Heteroarylgruppen sind Cyclopentadienyl-Anion-, Phenyl-, Naphthyl-, Pyrrolyl-, Imidazolyl-, Thiophenyl-, Pyridyl-, Pyrimidyl-, Indolyl-, Chinolinylreste.

Sowohl die Alkyl-, Alkenyl-, Alkinyl-, Aryl-, Heterocycloalkyl- und Heteroarylgruppen können Substituenten neben Wasserstoff auch (C₁-C₂₀)-Alkyl, (C₂-C₂₀)-Alkenyl, (C₁-C₁₀)-Haloalkyl, (C₃-C₈)-Cycloalkyl, (C₅-C₈)-Cycloalkenyl, (C₂-C₉)-Heterocycloalkyl, (C₁-C₉)-Heterocycloalkenyl, (C₅-C₁₄)-Aryl, (C₂-C₁₃)-Heteroaryl, wobei die Zahl der Heteroatome ausgewählt aus der Gruppe N, O, S, eins bis vier betragen kann, Fluor, Chlor, Brom, Iod, OH, NO₂, CF₃, O-Alkyl-(C₁-C₈), O-Aryl-(C₅-C₁₀), OCOH, OCO-Alkyl-(C₁-C₈), OCO-Alryl-(C₅-C₁₀), NH₂, NH(Alkyl-(C₁-C₈)), NH(Aryl-(C₅-C₁₀)), N(Alkyl-(C₁-C₈))₂, N(Aryl-(C₅-C₁₀))₂, NHCO-Alkyl-(C₁-C₈), NHCO-Aryl-(C₅-C₁₀), NHCOH, NHCOO-Alkyl-(C₁-C₄), NHCOO-Aryl-(C₅-C₁₀), CH(O-Alken-(C₂-C₈)-O), C(O-Alken-(C₂-C₈)-O)-Alkyl-(C₁-C₈), C(O-Alken-(C₂-C₈)-O)-Aryl-(C₅-C₁₀), CH(O-Alkyl-(C₁-C₈))₂, C(O-Alkyl-(C₁-C₈))₂-Alkyl-(C₁-C₈), C(O-Alkyl-(C₁-C₈))₂-Aryl-(C₅-C₁₀), CHO, CO-Alkyl-(C₁-C₈), CO-Aryl-(C₅-C₁₀), CN, COOH, COOM, COO-Alkyl-(C₁-C₈), COO-Aryl-(C₅-C₁₀), C(=NAlkyl-(C₁-C₈))O-Alkyl-(C₁-C₈), C(=NAryl-(C₅-C₁₀))O-Alkyl-(C₁-C₈), C(=NAlkyl-(C₁-C₈))O-Aryl-(C₅-C₁₀), C(=NAryl-(C₅-C₁₀))(O-Aryl-(C₅-C₁₀)), CONH₂, CONHAlkyl-(C₁-C₈), CON(Alkyl-(C₁-C₈))₂, CONHAryl-(C₅-C₁₀), CON(Aryl-(C₅-C₁₀))₂, CHCHCO₂H, CHCH-COOAlkyl-(C₁-C₈), P(Aryl-(C₅-C₁₀))₂, P(Alkyl-(C₁-C₈))₂, PO(Aryl-(C₅-C₁₀))₂, PO(Alkyl-(C₁-C₄))₂, PO₃H₂, PO₃M₂, POAlkyl-(C₁-C₄)(O-Alkyl-(C₁-C₆)), PO(O-Alkyl-(C₁-C₆))₂, OPO(Aryl-(C₅-C₁₀))₂, OPO(Alkyl-(C₁-C₄))₂, OPOH(Alkyl-(C₁-C₆)), OPO₃H₂, OPO₃M₂, OPOAlkyl-(C₁-C₄)(O-Alkyl-(C₁-C₆)), OPO(O-Alkyl-(C₁-C₆))₂, OPO(OAryl)₂-(C₅-C₁₀), SO₃H, SO₃M, SO₃-Alkyl-(C₁-C₄), SO₂-Alkyl-(C₁-C₆); SO-Alkyl-(C₁-C₆)), OSO₃H, OSO₃M, OSO₂-OAlkyl-(C₁-C₄), OSO₂-Alkyl-(C₁-C₆) oder Si(Alkyl-(C₁-C₈))₃, Si(C₁-C₈-Alkyl/C₅-C₁₀-Aryl)₃, wobei M ein Kation ausgewählt aus der Gruppe Li⁺, Na⁺, K⁺, Mg2⁺, Ca²⁺, NH₄⁺, N(C₁-C₁₀-Alkyl)₄⁺, N(H/C₁-C₁₀-Alkyl/C₅-C₁₀-Aryl)₄⁺ darstellt bedeuten, tragen.

Sowohl R¹ und R² als auch R³ und R⁴ können durch kovalente Bindungen verknüpft sein, so daß R¹ und R² als auch R³ und R⁴ jeweils eine 3 bis 15 Atome, bevorzugt 3 bis 8 Atome, aufweisende gesättigte oder ungesättigte carbocyclische Einheit oder eine entsprechende heterocyclische Einheit, mit ein bis vier Stickstoff-, Phosphor-, Silicium-, Schwefel- oder Sauerstoffatomen, bilden. Ferner ist durch eine kovalente Verknüpfung von R³/R¹ eine intramolekulare Reaktion unter Ringschluß möglich.

Besonders bevorzugte Substituenten R¹ und R² sind Wasserstoff, (C₁-C₁₂)-Alkyl, (C₂-C₁₂)-Alkenyl, (C₂-C₁₂)-Alkinyl, (C₅-C₁₀)-Aryl, CF₃, CHO, CO-Alkyl-(C₁-C₈), CO-Aryl-(C₅-C₁₀), CH(O-Alken-(C₂-C₄)-O), C(O-Alken-(C₂-C₄)-O)-Alkyl-(C₂-C₈), C(O-Alken-(C₂-C₄)-O)-Aryl-(C₅-C₁₀), CH(O-Alkyl-(C₁-C₈))₂, C(O-Alkyl-(C₁-C₈))₂-Alkyl-(C₁-C₈), C(O-Alkyl-(C₁-C₈))₂-Aryl-(C₅-C₁₀), CN, COOH, COOM, COO-Alkyl-(C₁-C₈), COO-Aryl-(C₅-C₁₀), C(=NAlkyl-(C₁-C₈))O-Alkyl-(C₁-C₈), C(=NAryl-(C₅-C₁₀))O-Alkyl-(C₁-C₈), C(=NAlkyl-(C₁-C₈))(O-Aryl-(C₅-C₁₀)), C(=NAryl-(C₅-C₁₀))O-Aryl-(C₅-C₁₀), CONH₂, CONHAlkyl-(C₁-C₈), CON(Alkyl-(C₁-C₈))₂, CONHAryl-(C₅-C₁₀), PO(Aryl-(C₅-C₁₀))₂, PO(Alkyl-(C₁-C₄))₂, POH(Alkyl-(C₁-C₆)), POH(Aryl-(C₅-C₁₀)), PO(Alkyl-(C₁-C₄))(O-Alkyl-(C₁-C₆)), PO(Alkyl-(C₁-C₆))(OAryl-(C₅-C₁₀)), PO(Aryl-(C₆-C₁₀))(OAlkyl-(C₁-C₆)), PO₃H₂, PO₃M₂, PO(O-Alkyl-(C₁-C₆))₂, PO(OAryl-(C₅-C₁₀))₂, PO(O-Alkyl-(C₁-C₆))(O-Aryl-(C₅-C₁₀)), SO₃H, SO₃M, SO₂-O-Alkyl-(C₁-C₄), SO₂-O-Aryl-(C₅-C₁₀), SO₂-Alkyl-(C₁-C₆), SO₂-Aryl-(C₅-C₁₀), SO-Alkyl-(C₁-C₈), SO-Aryl-(C₅-C₁₀) oder Si(Alkyl-(C₁-C₈))₃, Si(C₁-C₁₀-Alkyl/C₅-C₁₀-Aryl)₃, wobei M ein Kation ausgewählt aus der Gruppe Li⁺, Na⁺, K⁺, Mg²⁺, Ca^{2+,} NH₄⁺, N(C₁-C₁₀-Alkyl)₄⁺, N(C₁-C₁₀-Alkyl/C₅-C₁₀-Aryl)₄⁺ ist.

Besonders bevorzugte Substituenten R³ und R⁴ sind Wasserstoff, (C₁-C₁₂)-Alkyl, (C₂-C₁₂)-Alkenyl, (C₂-C₁₂)-Alkinyl, (C₅-C₁₀)-Aryl, O-Alkyl-(C₁-C₈), O-Aryl-(C₅-C₁₀), OH, NH₂, NH(Alkyl-(C₁-C₈)), N(Alkyl-(C₁-C₈))₂, NH(Aryl-(C₅-C₁₀)), NHCO-Alkyl-(C₁-C₄), NHCO-Aryl-(C₅-C₁₀),
wobei für die Reste R¹ bis R⁴ Alkyl einen nichtverzweigten oder verzweigten aliphatischen oder carbocyclischen Rest, Alkenyl einen olefinischen Kohlenwasserstoff, Alkinyl einen Acetylenkohlenwassersoff und Aryl einen aromatischen Rest darstellt. Dabei können ein bis vier Kohlenstoffatome des carbocyclischen und aromatischen Restes gegen ein bis vier Heteroatome aus der Gruppe der Stickstoff-, Sauerstoff- und Schwefelatome ausgetauscht sein.

Sowohl Alkyl, Alkenyl, Alkinyl, Aryl, Heterocycloalkyl und Heteroaryl können bevorzugt Substituenten die unabhängig voneinander Wasserstoff, (C₁-C₁₂)-Alkyl, (C₂-C₁₂)-Alkenyl, (C₁-C₁₀)-Haloalkyl, (C₃-C₈)-Cycloalkyl, (C₅-C₈)-Cycloalkenyl, (C₂-C₉)-Heterocycloalkyl, (C₁-C₉)-Heterocycloalkenyl, (C₅-C₁₀)-Aryl, (C₂-C₁₀)-Heteroaryl, wobei die Zahl der Heteroatome, bevorzugt aus der Gruppe N, O, S, eins bis vier betragen kann, Fluor, Chlor, Brom, Jod, OH, NO₂, CF₃, O-Alkyl-(C₁-C₈), O-Aryl-(C₅-C₁₀), OCO-Alkyl-(C₁-C₈), OCO-Aryl-(C₅-C₁₀), NH₂, NH(Alkyl-(C₁-C₈)), NH(Aryl-(C₅-C₁₀)), N(Alkyl-(C₁-C₈))₂, N(Aryl-(C₅-C₁₀))₂, NHCO-Alkyl-(C₁-C₈), NHCO-Aryl-(C₅-C₁₀), CH(O-Alken-(C₂-C₆)-O), C(O-Alken-(C₂-C₆)-O)-Alkyl-(C₁-C₈), C(O-Alken-(C₂-C₆)-O)-Aryl-(C₅-C₁₀), CH(O-Alkyl-(C₁-C₈))₂, C(O-Alkyl-(C₁-C₈))₂-Alkyl-(C₁-C₈), C(O-Alkyl-(C₁-C₈))₂-Aryl-(C₅-C₁₀), CHO, CO-Alkyl-(C₁-C₈), CO-Aryl-(C₅-C₁₀), CN, COOH, COOM, COO-Alkyl-(C₁-C₈), C(=NAlkyl-(C₁-C₈))O-Alkyl-(C₁-C₈), C(=NAryl-(C₅-C₁₀))O-Alkyl-(C₁-C₈), C(=NAlkyl-(C₁-C₈))O-Aryl-(C₅-C₁₀), C(=NAryl-(C₅-C₁₀))O-Aryl-(C₅-C₁₀), CONH₂, CO-Alkyl-(C₁-C₈), NHCOH, NHCOO-Alkyl-(C₁-C₄), CO-Aryl-(C₅-C₁₀), COO-Aryl-(C₅-C₁₀), CHCH-COOAlkyl-(C₁-C₈), CN, COOH, COOM, CHCHCO₂H, P(Aryl-(C₅-C₁₀))₂, P(Alkyl-(C₁-C₈))₂, PO(Aryl-(C₅-C₁₀))₂, PO(Alkyl-(C₁-C₄))₂, PO₃H₂, PO(Alkyl-(C₁-C₄))(O-Alkyl-(C₁-C₆)), PO(O-Alkyl-(C₁-C₆))₂, OPO(Aryl)₂-(C₅-C₁₀), OPO(Alkyl)₂-(C₁-C₄), OPOH(Alkyl-(C₁-C₆)), OPO₃H₂, OPO₃M₂, OPO(Alkyl-(C₁-C₄))(O-Alkyl-(C₁-C₆)), OPO(Aryl-(C₅-C₁₀))(O-Alkyl-(C₁-C₆)), OPO(O-Alkyl-(C₁-C₆))₂, OPO(OAryl-(C₅-C₁₀))₂, SO₃H, SO₃M, SO₂-O-Alkyl-(C₁-C₄), SO₂-O-Aryl-(C₅-C₁₀), SO₂-Alkyl-(C₁-C₆), SO₂-Aryl-(C₅-C₁₀), SO-Alkyl-(C₁-C₆)), SO-Aryl-(C₅-C₁₀), OSO₃H, OSO₃M, OSO₂-OAlkyl-(C₁-C₄), OSO₂-Alkyl-(C₁-C₆) oder Si(Alkyl-(C₁-C₈))₃, Si(C₁-C₈-Alkyl/C₅-C₁₀-Aryl)₃, wobei M ein Kation aus der Gruppe Li⁺, Na⁺, K⁺, Mg²⁺, Ca²⁺, NH₄⁺, N(C₁-C₁₀-Alkyl)₄⁺, N(H/C₁-C₁₀-Alkyl/C₆-C₁₀-Aryl)₄⁺ darstellt, bedeuten, tragen.

Besonders bevorzugt sind Carbonylverbindungen (II), bei denen Reste R¹ und R² verschieden sind und somit ein enantiomerenreines oder enantiomerenangereichertes Amine (III) synthetisiert werden kann.

Bei den Substituenten der Arylgruppen kann es sich auch um π-gebundenen Metallkomplexen der allgemeinen Formeln (IV-V) handeln, 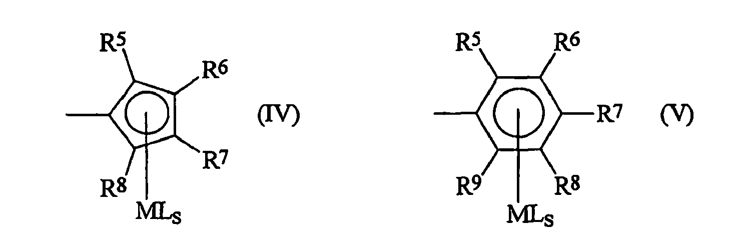 worin s ganze Zahlen im Bereich von 1 bis 6 und M Chrom, Molybdän, Wolfram, Mangan, Rhenium, Eisen, Kobalt, Nickel oder ein Element der Lantanidenreihe bedeuten und R⁵ bis R⁹ gleich oder verschieden sind und einem der für R³-R⁴ definierten Reste entsprechen und worin die Liganden L₁ bis L_{S} gleich oder verschieden sind und für ein cyclischen Äther mit 5-6 Ringatomen, ein cyclisches Olefin mit 5-8 Ringatomen, Pyridin, CO, PF₃ oder ein Ligand der allgemeinen Formeln (VI) und (VII) stehen.

Die erfindungsgemäße Umsetzung der Ketone oder Aldehyde zu den Aminen gelingt überraschend effizient durch eine reduktive Hydridotransfer-Aminierung der Carbonylverbindung in Gegenwart von katalytisch wirkenden Übergangsmetallkomplexen enthaltend mindestens ein Metall aus der Gruppe Fe, Ru, Os, Co, Rh, Ir, Ni, Pd, Pt und mindestens einen mono- oder bidentate Stickstoffdonorliganden, Phosphordonorliganden, Cyclopentadienylliganden, Arenliganden, Olefinliganden, Alkinliganden, Heterocycloalkylliganden, Heteroarylliganden, Hydridliganden, Alkylliganden und/oder Carbonylliganden enthalten.

Prinzipiell sind alle Liganden geeignet, die stabile Komplexverbindungen mit Metallen der achten Nebengruppe eingehen.

Beispiele für besonders geeignete Stickstoff-, Phosphor-, Cyclopentadienyl- und Aren- Liganden sind mono- oder bidentate Liganden der Formel (VI) oder (VII),

L¹-R¹² (VI)

L¹-Q-L² (VII)

worin, L¹ und L² unabhängig voneinander für eine koordinierende Gruppe der Formeln (VIII) - (XII) stehen 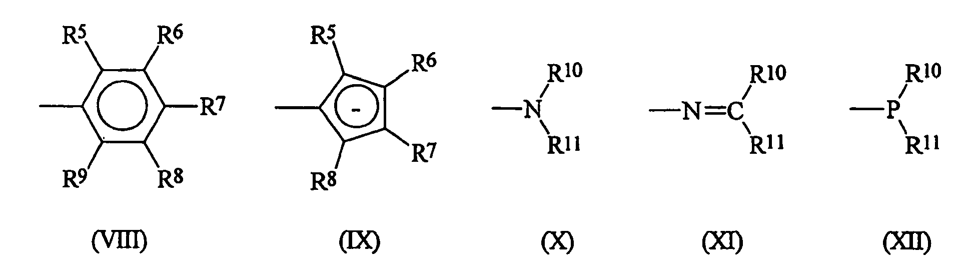 wobei R⁵ bis R⁹ unabhängig voneinander aus der Gruppe Wasserstoff, (C₁-C₂₄)-Alkyl, (C₂-C₂₄)-Alkenyl, (C₂-C₂₄)-Alkinyl, (C₅-C₁₀)-Aryl, CF₃, CO-Alkyl-(C₁-C₈), CO-Aryl-(C₅-C₁₀), CN, COOH, COOM, COO-Alkyl-(C₁-C₈), COO-Aryl-(C₅-C₁₀), C(=NAlkyl-(C₁-C₈))O-Alkyl-(C₁-C₈), C(=NAryl-(C₅-C₁₀))(O-Alkyl-(C₁-C₈)), C(=NAlkyl-(C₁-C₈))(O-Aryl-(C₅-C₁₀)), C(=NAryl-(C₅-C₁₀))(O-Aryl-(C₅-C₁₀)), CONH₂, CONHAlkyl-(C₁-C₈), CON(Alkyl-(C₁-C₈))₂, CONHAryl-(C₅-C₁₀), CON(Aryl-(C₅-C₁₀))₂, PO(Aryl-(C₅-C₁₀))₂, PO(Alkyl-(C₁-C₄))₂, POH(Alkyl-(C₁-C₆)), POH(Aryl-(C₅-C₁₀)), PO(Alkyl-(C₁-C₄))(O-Alkyl-(C₁-C₆)), PO(Alkyl-(C₁-C₆))(OAryl-(C₅-C₁₀)), PO(Aryl-(C₅-C₁₀))(OAlkyl-(C₁-C₆)), PO₃H₂, PO₃M₂, PO(O-Alkyl-(C₁-C₆))₂, PO(OAryl-(C₅-C₁₀))₂, PO(O-Alkyl-(C₁-C₆))(O-Aryl-(C₅-C₁₀)), SO₃H, SO₃M, SO₃-Alkyl-(C₁-C₄), SO₃-Aryl-(C₅-C₁₀), SO₂-Alkyl-(C₁-C₆), SO₂-Aryl-(C₅-C₁₀), SO-Alkyl-(C₁-C₈), SO-Aryl-(C₅-C₁₀), S-Alkyl-(C₁-C₈), S-Aryl-(C₅-C₁₀), SH, Si(Alkyl-(C₁-C₈))₃, Si(C₁-C₁₀-Alkyl/C₅-C₁₀-Aryl)₃, NO₂, F, Cl, Br, I, O-Alkyl-(C₁-C₈), O-Aryl-(C₅-C₁₀), OH, NH₂, NH(Alkyl-(C₁-C₈)), N(Alkyl-(C₁-C₈))₂, NH(Aryl-(C₅-C₁₀)), NHCO-Alkyl-(C₁-C₄), NHCO-Aryl-(C₅-C₁₀), NHCOO-Alkyl-(C₁-C₄), NHCOO-Aryl-(C₅-C₁₀), OCO-Alkyl-(C₁-C₈), OCO-Aryl-(C₅-C₁₀), OPO(Aryl-(C₅-C₁₀))₂, OPO(Alkyl-(C₁-C₄))₂, OPOH(Alkyl-(C₁-C₆)), OPO₃H₂, OPO₃M₂, OPOAlkyl-(C₁-C₄)(O-Alkyl-(C₁-C₆)), OPO(O-Alkyl-(C₁-C₆))₂, OPO(OAryl-(C₅-C₁₀))₂, OSO₃H, OSO₃M, OSO₂-CF₃, OSO₃-Alkyl-(C₁-C₄), OSO₃-Aryl-(C₅-C₁₀), OSO₂-Alkyl-(C₁-C₆), OSO₂-Aryl-(C₅-C₁₀), wobei M ein Kation Li⁺, Na⁺, K⁺, Mg²⁺, Ca²⁺, NH₄⁺, N(C₁-C₁₀-Alkyl)₄⁺, N(C₁-C₁₀-Alkyl/C₅-C₁₀-Aryl)₄⁺ darstellt, ausgewählt sind,
und wobei die Reste R¹⁰ bis R¹² unabhängig voneinander einen Wasserstoff, (C₁-C₂₄)-Alkyl, (C₂-C₂₄)-Alkenyl, (C₂-C₂₄)-Alkinyl, C₅-C₈ Cycloalkenyl, (C₅-C₁₄)-Aryl, O-Alkyl-(C₁-C₈), O-Aryl-(C₅-C₁₄), O-Alkenyl-(C₂-C₂₄), O-Alkinyl-(C₂-C₂₄), O-Cycloalkenyl-(C₅-C₈), O-Aryl-(C₅-C₁₄), F, NH₂, NH(Alkyl-(C₁-C₈)), NH-Alkenyl-(C₂-C₂₄), NH-Alkinyl-(C₂-C₂₄), NH-Cycloalkenyl-(C₅-C₈), NH-Aryl-(C₅-C₁₄), N(Alkyl-(C₁-C₈))₂, N(Alkenyl-(C₂-C₂₄))₂, N(Alkinyl-(C₂-C₂₄))₂, N(Cycloalkenyl-(C₅-C₆))₂, N(Alkyl-(C₁-C₈))(Aryl-(C₅-C₁₀)), N(Aryl-(C₅-C₁₀))₂, NHCO-Alkyl-(C₁-C₄), NHCO-Alkenyl-(C₂-C₂₄), NHCO-Alkinyl-(C₂-C₂₄), NHCO-Cycloalkenyl-(C₅-C₈), NHCO-Aryl-(C₅-C₁₄), OCO-Alkyl-(C₁-C₄), OCO-Alkenyl-(C₂-C₂₄), OCO-Cycloalkenyl-(C₅-C₈), OCO-Aryl-(C₅-C₁₄), SO₂-Alkyl-(C₁-C₆), SO₂-Aryl-(C₅-C₁₀)-Rest darstellen können und
bei denen alle obengenannten Substituenten jeweils ein- oder mehrfach substituiert sein können, wobei die cyclischen aliphatischen oder aromatischen Reste bevorzugt 5 bis 7 gliedrige Ringe sind, wobei gegebenenfalls auch zwei benachbarte Reste gemeinsam für 3 bis 15 Atome aufweisende gesättigte oder ungesättigte carbocyclische oder heterocyclische Gruppen, mit ein bis vier Stickstoff-, Phosphor-, Silicium-, Schwefel- oder Sauerstoffatom, stehen können. Unter Substituenten der Cyclopentadienyl- und Arylgruppen sind auch π- gebundene Metall-Komplexe der allgemeinen Formeln (IV) oder (V) zu verstehen.

Q stellt eine Brücke, die die Einheit L¹ mit der Einheit L² verbindet, der Formel (XIII) dar:

X¹-Z-X² (XIII)

wobei X¹ und X² unabhängig voneinander eine direkte Bindung oder eine Gruppe -O-, -S-, oder -NR¹³- darstellt, wobei R¹³ einem der für R¹⁰-R¹² definierten Reste sein kann und wobei
Z eine direkte Bindung oder aus durch Einfach- oder Mehrfachbindungen verknüpfte 1-16 Kohlenstoffatome sein können, wobei ein bis vier Kohlenstoffatome durch Heteroatome, bevorzugt aus der Gruppe N, O, S oder Si ersetzt sein können, so dass z. B. ein aliphatisches, heteroaliphatisches, cyloaliphatisches, heterocycloaliphatisches olefinisches, heteroolefinisches, acetylenisches, heteroacetylenisches, cycloolefinischen, heterocycloolefinisches, aromatisches oder heteroaromatisches Systems oder ein Metallocen vorliegt, welches mit Substituenten wie für R⁵-R⁹ angegeben ein oder mehrfach substituiert sein kann oder einen Keto-(=O), Thioketo- (=S) oder Imidsubstituenten (=NR¹⁴) tragen kann, wobei R¹⁴ einer der für R¹⁰-R¹² definierten Reste sein kann
und worin die Einheiten L¹ und R¹² oder L¹ und Q oder L² und Q so miteinander verknüpft sein können, dass ein 3 bis 15 Atome aufweisendes gesättigtes oder ungesättigtes carbocyclisches oder ein gesättigtes oder ungesättigtes ein bis vier Stickstoff-, Phosphor-, Silicium-, Schwefel- oder Sauerstoffatome enthaltendes, heterocyclisches Gerüst entsteht.

In einer bevorzugt Ausführungsform stellt Q in Formel (VII) eine aus ein bis vierzehn Kohlenstoffatomen bestehende aliphatische, olefinische oder acetylenische Brücke dar, wobei ein bis vier Kohlenstoffatome gegen Stickstoff- oder Siliciumatome oder ein bis zwei Kohlenstoffatome gegen Sauerstoff- oder Schwefelatome ausgetauscht sein können und wobei die einzelnen Brückenglieder der Gruppe unabhängig voneinander Substituenten tragen können wie sie für R⁵ - R⁹ definiert sind oder einen Keto- (=O), Thioketo- (=S) oder Imidsubstituenten (=NR¹⁴) tragen können, wobei R¹⁴ einem der für R¹⁰-R¹² definierten Reste sein kann und worin zwei der Einheiten L¹, L² und Q miteinander verknüpft sein können, so dass ein 5 bis 9 Atome aufweisendes gesättigtes oder ungesättigtes carbocyclisches oder ein gesättigtes oder ungesättigtes ein bis vier Stickstoff-, Phosphor-, Silizium-, Schwefel- oder Sauerstoffatome enthaltendes, heterocyclisches Gerüst entsteht.

Beispiele für Olefin- und Alkinliganden sind mono-, bi-, tri- oder tetradentate lineare, verzweigte oder polycyclische, insbesondere mono- und bicyclische, Alkine, Olefine, konjugierte oder nicht konjugierte Di- Tri- oder Tetraene mit zwei bis zwölf Kohlenstoffatomen, wobei ein bis vier Kohlenstoffatome gegen Stickstoff- oder Siliciumatome oder ein bis zwei Kohlenstoffatome gegen Sauerstoff- oder Schwefelatome ausgetauscht sein können und wobei die einzelnen Glieder der Gruppe unabhängig voneinander Substituenten tragen können wie sie für R⁵ - R⁹ definiert sind oder einen Keto- (=O), Thioketo- (=S) oder Imidsubstituenten (=NR¹⁴) tragen können, wobei R¹⁴ einem der für R¹⁰-R¹² definierten Reste sein kann.

Beispiele für Heterocycloalkyl- oder Heteroarylliganden sind mono- oder bidentate polycyclische, insbesondere mono- und bicyclische gemeinsam für 3 bis 15 Atome aufweisende gesättigte oder ungesättigte heterocyclische Liganden , mit ein bis vier Sauerstoff-, Schwefel- oder Stickstoffatome oder (C₃-C₁₃)-Heteroaromaten, mit ein bis vier Sauerstoff-, Schwefel-, Stickstoff- oder ein bis zwei Phosphoratome, wobei die einzelnen Brückenglieder unabhängig voneinander Substituenten tragen können wie sie für R⁵ - R⁹ definiert sind oder einen Keto- (=O), Thioketo- (=S) oder Imidsubstituenten (=NR¹⁴) tragen können, wobei R¹⁴ einem der für R¹⁰-R¹² definierten Reste sein kann.

Bevorzugte Liganden sind Liganden der allgemeinen Formeln (VI) und (VII), unter denen wiederum solche bevorzugt sind, bei denen R⁵ bis R¹² unabhängig voneinander C₁-C₈-Alkyl, C₅-C₆-Cycloalkyl, C₆-Aryl, C₄-C₅-Heteroaryl, wobei die Zahl der Heteroatome 1-2 beträgt, bevorzugt ausgewählt aus der Gruppe N, O, S und die Ringgröße 5-6 beträgt, Naphtyl, O-Alkyl-(C₁-C₈), O-Aryl-C₆, O-Cycloalkenyl-(C₅-C₈), O-Aryl-C₆, O-Phenyl, O-Naphthyl, F, NH₂, NH(Alkyl-(C₁-C₈)), NH-Cycloalkenyl-(C₅-C₈), NH-Phenyl, NH-Naphthyl, N(Alkyl-(C₁-C₈))₂, N(Cycloalkenyl-(C₅-C₈))₂, N(Alkyl-(C₁-C₈))(Aryl-C₆), N(Aryl-C₆)₂ sind, dabei können diese Gruppen und koordinierende Cyclopentadienyl- und Aryl-Gruppen der allgemeinen Formeln (VIII-IX) eine oder mehrere Substituenten tragen, bevorzugt sind Substituenten, die unabhängig voneinander Wasserstoff, C₁-C₁₀ Alkyl, C₁-C₆ Haloalkyl, C₅-C₆ Cycloalkyl, C₂-C₉ Heterocycloalkyl, Phenyl, C₄-C₅ Heteroaryl, wobei die Zahl der Heteroatome, bevorzugt aus der Gruppe N, O, S, 1-2 betragen kann, C₁-C₆ Alkoxy, OCO-Alkyl-(C₁-C₆), O-Aryl-C₆, Trihalomethyl, Fluoro, Chloro, Bromo, Iodo, Nitro, Hydroxy, Oxo, Thio, Thiolato, Amino, C₁-C₈ substituierte Amino der Formen mono-, di-, tri- C₁-C₈-Alkylamino oder C₂-C₈ Alkenylamino oder mono- und di-C₆-C₈ Arylamino oder C₁-C₈-Alkyl-C₆-C₈-arylamino, NH-CO-Alkyl- C₁-C₈, NH-CO-Aryl- C₆-C₈, C₁-C₈-Acyloxy, Carboxyl, Carboxylato der Form COOR¹⁴, Sulfinato, Sulfonato der Form SO₃R¹⁴, Phosphonato der Form PO₃H₂, PO₃HR¹⁴, PO₃R¹⁴₂, wobei R¹⁴ entweder ein ein- oder zweiwertiges Kation (Li⁺, Na⁺, K⁺, Mg²⁺, Ca²⁺), NH₄⁺, N(C₁-C₁₀-Alkyl)₄⁺, N(H/C₁-C₁₀-Alkyl/ C₆-C₁₀-Aryl)₄⁺, C₁-C₈-Alkyl oder C₆-Aryl darstellt, Tri- C₁-C₆ Alkylsilyl, sein können.

Bevorzugt sind weiterhin Liganden, bei denen Q aus ein bis vier Kohlenstoffatomen und einem Sauerstoff- oder Stickstoffatom, besonders bevorzugt aus zwei Kohlenstoffatomen, besteht. Ist Q Teil eines cyclischen Strukturelementes, sind drei bis neungliedrige Ringsysteme bevorzugt. Besonders bevorzugt sind fünf bis siebengliedrige Ringsysteme. Das Ringsystem kann ein bis vier Heteroatome enthalten, bevorzugt ein bis zwei. Bevorzugte Heteroatome sind O, N und S. Der Stickstoff des Ringsystems kann als NR¹³, NR¹³R¹³⁺, NR¹³H⁺, NC(O)R¹³, vorliegen. Die Ringsysteme können wie für R⁵ bis R⁹ angegeben ein oder mehrfach direkt substituiert sein, wobei die Substituenten auch untereinander verbrückt sein können.

Besonders bevorzugte Ringsysteme sind unsubstituierte oder wie vorstehend angegeben substituierte Phenyl, Ferrocenyl, Cyclopentyl, Cyclohexyl, Pyridyl, Pyrrol, Furyl, Thiophen, Tetrahydrofuran, Tetrahydrothiophen, Piperidyl, Pyrrolidinyl, Dioxolan oder Sulfolanringe.
Metallocene wie Ferrocene und Aren-Komplexe wie Benzolchromtricarbonyl werden im Sinne dieser Erfindung zur Gruppe der Aromaten gerechnet.

Beispiele für besonders geeignete achiralen oder chiralen Liganden stellen Verbindungen der Formeln dar: 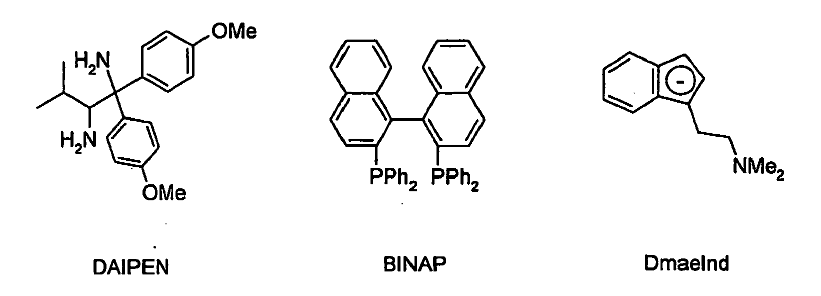 Weitere bevorzugt verwendbare chirale und achirale Phosphan und Diphosphan-Liganden sind z. B. das dppb (1,4-Bis(diphenylphosphino)butan), dcypb (1,4-Bis(dicyclohexylphosphino)butan), (R,R)-DIPAMP ((1 R,2R)-Bis [(2-methoxyphenyl) phenylphosphino]ethan); (R)-Norphos ((2R,3R)-(-)-2,3-Bis(diphenylphosphino)-bicyclo[2.2.1]hept-5-en); (R,R)-CHIRAPHOS ((2R,3R)-(-)Bis(diphenylphosphino)butan) (S.H. Bergens, J. Whelan, B. Bosnich Inorg. Synth. (1997), 31, 131-138); (R,R)-DEGUPHOS ((3R,4R)-(+)-1-Benzyl-3,4-bis(diphenylphosphino)-pyrrolidin); (R)-CyGanterPhos ((R)-[3,4-Dimethylphosphaferrocen-2-yl)methyl]-dicyclohexylphosphan); (R,R)-Me-DUPHOS ((-)-1,2-Bis((2R,5R)-2,5-dimethylphospholano)benzen); (R,R)-Et-DUPHOS ((-)-1,2-Bis((2R,5R)-2,5-diethylphospholano)benzen); (R,R)-Me-BPE ((+)-1,2-Bis((2R,5R)-2,5-dimethylphospholano)ethan); (R,R)-Et-BPE ((+)-1,2-Bis((2R,5R)-2,5-diethylphospholano)ethan); (R)-Bis(MePheP)benzol ((1R,2R)-(+)-Bis-(methylphenylphosphino)benzen); (R)-PROPHOS (2-(R)-1,2-bis(diphenylphosphino)propan); (R,R)-SKEWPHOS ((2R,4R)-(-)Bis-(diphenylphosphino)pentan) (P.A. MacNeil, N.K. Roberts, B. Bosnich J. Am. Chem. Soc. (1981), 103, 2273-80); (S)-Phos4 ((S)-1-[2'-(Diphenylphosphino)-phenyl]diphenylphosphinoethan; (R,S)-Cy-Fc-etdpp ((R)-1-{(1S)-2-(Dicyclohexylphosphino)ferrocenyl}ethyldiphenylphosphin; (R,S)-Cy-Fc-etdCyP ((R)-1-{(1S)-2-(Dicyclohexylphosphino)ferrocenyl}ethyldicyclohexylphosphin); (R,S)-Ph-Fc-etdtBuP ((R)-1-{(1S)-2-(Diphenylphosphino)ferrocenyl}ethyldi-tert-butylphosphin); (R,S)-JOSIPHOS ((R)-1-{(1S)-2-(Diphenylphosphino)ferrocenyl}-ethyldicyclohexylphosphin) (EP 564406); (R)-Carboxybutyl-BINAP ((R)-(+)-7-Carboxybutyl-2,2'-bis(diphenylphosphino)-1,1'-binaphtyl); (*R*)-BINAP ((*R*)-2,2'-Bis(diphenylphosphino)-1,1'-binapthyl) (A. Miyashita, A. Yasuda, H. Takaya, K. Toriumi, T. Ito, T. Souchi, R. Noyori, R. J. Am. Chem. Soc. (1980), 102, 7932-4); (*R*)-Tol-BINAP ((*R*)-2,2'-Bis(di-p-tolylphosphino)-1,1'-binapthyl) (H. Takaya, K. Mashima, K. Koyano, M. Yagi, H. Kumobayashi, T. Taketomi, S. Akutagawa, R Noyori J. Org. Chem. (1986), 51, 629-35); (R)-MeO-BIPHEP ((R)-(-)-2,2'-Bis(diphenylphosphino)-6,6'-dimethoxy-1,1'-biphenyl); (R)-p-Tol-MeO-BIPHEP ((R)-(-)-2,2'-Bis(di-p-tolylphosphino)-6,6'-dimethoxy-1,1'-biphenyl); (S,S)-1,2-(BDPPmethyl)-cyclohexan ((1S,2S)-(+)-trans-1,2-Bis(diphenylphosphinomethyl)cyclohexan); (S,S)-DIOP (4S,5S- (+)-1,4-Bis(diphenylphosphino)-1,4-dideoxy-2,3-isopropyliden-D-threitol) (Kagan et al. J. Amer. Chem. Soc. (1972), 94, 6429); (S)-MOD-DIOP (S,S-(+)-1,4-Bis[bis(3,5-dimethyl-4-methoxyphenylphosphino)]-1,4-dideoxy-2,3-isopropyliden-D-threitol); (*R*)-MeAAPHOS ((2*R*,3*R*,5*R*,6*R*)-2,3-Dimethoxy-2,3-dimethyl-5,6-bis(diphenylphosphinomethyl)-1,4-dioxan) (Berens et al. J. Org. Chem. (1995), 60, 8204), (S,S)-BPPM-H ((2S,4S)-(-)-4-Diphenylphosphino-2-(diphenylphosphinomethyl)pyrrolidin); (S,S)-BPPM ((2S,4S)-N-tert-Butoxycarbonyl-4-(diphenylphosphino)-2-(diphenylphosphinomethyl)pyrrolidin) (I. Ojima, T. Kogure, N. Yoda J. Org. Chem. (1980), 45, 4728-39); (R,R)-Phenyl-CAPP ((2R,4R)-N-Anilidooxy-4-(diphenylphosphino)-2-(diphenylphosphinomethyl)pyrrolidin); (R)-NAPHOS ((R)-(+)-2,2'-Bis(diphenylphosphinomethyl)-1,1'-binaphtyl); Diphosphinit-Liganden auf Kohlenhydratbasis wie z. B. Ph-β-Glup (Phenyl 4,6-O-benzyliden-2,3-bis(O-diphenylphosphino)-β-D-glucopyranosid) oder Ph-β-Glup-OH (Phenyl 2,3-Bis(O-diphenylphosphino)-β-D-glucopyranosid) in DD 140036 und WO 95/18787 beschrieben und verwandte Ligandsysteme wie z. B. das DPOE (1,2-Bis(diphenylphosphinoxy)ethan) (S. Bolano, J. Bravo, R. Carballo, S. Garcia-Fontan, U. Abram, E.M. Vazquez-Lopez Polyhedron (1999), 18, 1431-1436); (*R*,*R*)-bdpch ((1*R*,2*R*)-(trans)-1,2-Bis-(diphenylphosphinoxy)cyclohexan) (M. Tanaka, I. Ogata J. Chem. Soc., Chem. Commun. (1975), 735); (R,R)-CYLOPP-2-Me ((1*R*,2*R*)-(trans)-1,2-Bis-(di-(2-methylphenyl)phosphinoxy)cyclohexan); (R,R)-CYCLOPP-4-CF3 ((1*R*,2*R*)-(trans)-1,2-Bis-(di-(4-trifluormethylphenyl)phosphinoxy)cyclohexan); (R,R)-CYCLOPP-3,5-CI ((1*R*,2*R*)-(trans)-1,2-Bis-(di-(3,5-dichlorphenyl)-phosphinoxy)cyclohexan); (R,R)-CYCLOPP-3,5-CF3 ((1*R*,2*R*)-(trans)-1,2-Bis-(di-(3,5-bis-trifluormethyl)phenyl)phosphinoxy)cyclohexan); (R,R)-CYCLOPP-3,5-F ((1*R*,2*R*)-(trans)-1,2-Bis-(di-(3,5-difluorphenyl)phosphinoxy)cyclohexan); CARBOPHOS-3,5-Me2Ph (Methyl 2,6-(O)-dibenzoyl-3,4-(O)-bis-(bis-(3,5-dimethylphenyl)phosphino)-α-D-glucopyranosid); GLUCOPHOS-Ph-3,5-Me (Phenyl-4,6-O-(R)-benzyliden-2,3-(O)-bis(bis-(3,5-dimethylphenyl)phosphino)-β-D-glucopyranosid); *R*-POP-Bz ((3R,4R)-3,4-diphenylphosphinoxy-1-benzylpyrrolidin) (M. Yatagai, T. Yamagishi, M. Hida Bull. Chem. Soc. Jpn. (1984), 57, 823-6); (R,S)-Phos3 ((3*R*,4*S*)-3-Diphenylphosphinoxy-4-[4-(di(4-fluorophenyl)phosphino-2,5-dimethyl-3-thienyl]tetrahydrofuran) und Aminophosphin-Phosphinite (Agbossou et al., Coordination Chemistry Rev. 1998, 178-180, 1615), wie z.B. (S)-CyCy-OxoPRONOP ((*S*)-1-(Dicyclohexylphosphino)-2-(dicyclohexylphosphinoxymethyl)-pyrrolidon-5); (S)-Cy,Cy-PRONOP ((*S*)-1-(Dicyclohexylphosphino)-2-(dicyclohexylphosphinoxymethyl)pyrrolidin); (S)-CyCyisoALANOP ((*S*)-2-(N-Methyl-N-dicyclohexylphosphino)amino-1-(dicyclohexylphosphinoxy)propan); (R)-PROPRAPHOS ((*R*)-2-(N-tsopropyl-N-diphenylphosphino)amino-1-(diphenylphosphinoxy)propan) und PROPRAHOS-Analog (*R*)-Cyp-PPP ((*2R*)-1-[[(diphenylphosphino)(cyclopenthyl)amino]methyl]-2-diphenylphosphinoxy-3-(1-naphthalenyloxy)propan) (Krause et al. J. Mol. Catal. A: Chem. (1995), 104, 147), Aminophosphane, wie z.B.: (R)-PN-Ph ((R)-(-)-2-[2-(Diphenylphosphino)-phenyl]-4-phenyl-1,3-oxazolin); (S)-PN-iPr ((S)-(+)-2-[2-(Diphenylphosphino)-phenyl]-4-isopropyl-1,3-oxazolin); (S)-PN-iPr-Me ((S)-(+)-2-[2-(Diphenylphosphino)-phenyl]-4-(isopropyl)-methyl-1,3-oxazolin); (S)-PN-tBu ((S)-(+)-2-[2-(Diphenylphosphino)-phenyl]-4-(2-methyl)-isopropyl-1,3-oxazolin) (Koch G., Lloyd-Jones G. C., Loiseleur O., Pfaltz A., Pretot R., Schaffner S., Schnider P., von Matt P. Recl. Trav. Chim. Pays-Bas 1995, 114, 206-10); (R)-QUINAP ((R)-(+)-1-(2-Diphenylphosphino-1-naphthyl)-isoquinoline); (R,R)-(S,S)-EtTRAP ((*R*,*R*)-2,2"-Bis[(*S*)-1-(diethylphosphino)ethyl]-1,1"-bisferrocen). Die phosphorhaltigen Liganden lassen sich gemäß dem Fachmann bekannten Syntheseverfahren, wie sie z. B. nach Methoden wie sie in Chemistry of Organophosphorous Compounds, Ed. F. R. Hartley, Serial Ed. S. Patai, Vol. 1, John. Whiley, 1990 beschrieben sind, herstellen. Einige der Liganden und Metallkomplexe sind auch käuflich erhältlich.

Neben den bevorzugten Liganden eignen sich darüber hinaus auch mono- oder bidentate lineare verzweigte oder cyclische, insbesondere mono- und bicyclische, olefinische Liganden oder Alkine mit zwei bis zwölf Kohlenstoffatomen oder (C₃-C₁₃)-Heteroaromaten, mit ein bis vier Stickstoff- oder ein bis zwei Posphoratome. Beispiele für olefinische Liganden sind Ethylen, Butadien, Cyclohexen, 1,3-Cyclohexadien, cis-Cyloocten, 1,5-cis,cis-Cyclooctadien, Norborbornen, Norbornadien. Beispiele für Heteroaromaten sind Pyridin, 2,2'-Bipyridyl, Phosphabenzol und Phosphaferrocen.

Die Herstellung der katalytisch aktiven Metall-Ligand-Komplexverbindungen kann in situ durch Reaktion eines Metallsalzes oder eines entsprechenden Vorkomplexes mit den Liganden der allgemeinen Formeln (VI) und/oder (VII) erfolgen. Darüber hinaus kann eine Metall-Ligand-Komplexverbindung durch Reaktion eines Metallsalzes oder eines entsprechenden Vorkomplexes mit den Liganden der allgemeinen Formel (VI) und/oder (VII) und anschließende Isolierung gewonnen werden.

Die Metallkomplexe lassen sich beispielsweise synthetisieren, indem in bekannter Weise (EP-A -0158875; EP-A-0437690) z. B. durch Umsetzung mit Eisen-, Rhodium-, Osmium-, Iridium-, Ruthenium-, Palladium-, Platin-, Cobalt- bzw. Nickelkomplexen, die labile Liganden enthalten (z.B. [Rh(COD)₂]BF₄, [RuCl₂(C₆H₆)]₂, [RuCl₂(C₅Me₅)]₂, [Ir(COD)Cl]₂) mit den achiralen oder chiralen Liganden der allgemeinen Formeln (VI) und/oder (VII) katalytisch aktive Komplexe generiert. Weitere Verfahren zur Herstellung von Metallkomplexen sind in Comprehensive Organometallic Chemistry II, Pergamon 1995 beschrieben. Die Katalysatoren
können unmittelbar vor Reaktion aus dem Metallprecursor und den Liganden Formel (VI) und/oder (VII) in situ oder in einem inerten Lösungsmittel erzeugt werden, oder sie werden in isolierter Form eingesetzt. Viele der Katalysatoren bzw. der einsetzbaren Liganden und Vorkomplexe sind auch kommerziell erhältlich.

Beispiele für einsetzbare Metallsalzedukte sind deren Chloride, Bromide, Iodide,-Nitrate, Acetate, Acetylacetonate, Hexafluoracetylacetonate, Perfluoracetate oder-Triflate.

### Beispiele für geeignete Vorkomplexe sind:

Bis(cycloocten)rhodium(I)chlorid-Dimer, 1,5-Cyclooctadienrhodium(I)chlorid-Dimer, Norbornadienrhodium(I)chlorid-Dimer, 1,5-Hexadienrhodium(I)chlorid-Dimer, Tris(triphenylphosphan)rhodium(I)chlorid, Hydridocarbonyltris(triphenylphosphan)rhodium(1)chlorid, Bis(1,5-cyclooctadien)rhodium(I)perchlorat, Bis(1,5-cyclooctadien)rhodium(I)tetrafluorborat, Bis(1,5-cyclooctadien)rhodium(I)triflat, Bis(acetonitril)(1,5-cyclooctadien)rhodium(I)perchlorat, Bis(acetonitril)(1,5-cyclooctadien)rhodium(I)tetrafluorborat, Bis(acetonitril)(1,5-cyclooctadien)rhodium(I)triflat, Cyclopentadienrhodium(III)chlorid-Dimer, Pentamethylcyclopentadienrhodium(III)chlorid-Dimer, Cyclooctadieniridium(I)chlorid-Dimer, Bis(cycloocten)iridium(I)chlorid-Dimer, Bis(2,2,6,6-tetramethyl-3,5-heptanedionato)(1,5-cyclooctadien)ruthenium (II), Carbonyl(dihydrido)tris(triphenylphosphin)ruthenium (II), Chloro(cyclopentadienyl)bis(triphenylphosphin)ruthenium (II), Chloro(indenyl)bis(triphenylphosphin)ruthenium (II), cis-Dichlorobis(2,2'-bipyridin)ruthenium (II) dihydrat, Dichloro(1,5-cyclooctadien)ruthenium (II), Dichlorodicarbonylbis(triphenylphosphin)ruthenium (II), Dichloro(pentamethylcyclopentadienyl)ruthenium (III), Dichlorotris(triphenylphosphin)ruthenium (II), Tris(2,2'-bipyridyl)ruthenium (II)chlorid, Benzenruthenium(II)chlorid-Dimer, Dichlor(*p*-cymen)ruthenium(II)-Dimer, (Bicyclo[2.2.1]hepta-2,5-dien)dichlororuthenium(II), Bis(2-methylallyl)-1,5-cyclooctadien-ruthenium

Bei der Umsetzung der Liganden der Formeln (VI) und (VII) mit den Metallvorkomplexen können z. B. Komplexverbindungen vom Typ (XIV) erhalten werden, die als Katalysatoren bei der erfindungsgemäßen reduktiven Hydridotransfer-Aminierung wirken können,

(MₓL'ₘL"ₙS_{q}]Aᵣ (XIV)

wobei in der allgemeinen Formel (XIV) M ein, bevorzugt einkerniges, Fe, Ru, Os, Co, Rh, Ir, Ni, Pd oder Pt Metallzentrum darstellt, L" gleiche oder verschiedene koordinierende organische oder anorganische Liganden aus dem Vorkomplex und L' organische Liganden der Formeln (VI) und/oder (VII) darstellen, S koordinierende Lösungsmittelmoleküle und A Äquivalente aus nicht koordinierenden Anionen repräsentiert, wobei x und m ganzen Zahlen größer oder gleich 1, n, q und r ganze Zahlen größer oder gleich 0 sind.

Die Summe m + n + q wird durch die an den Metallzentren zur Verfügung stehenden Koordinationszentren nach oben begrenzt, wobei nicht alle Koordinationsstellen besetzt sein müssen.

Bevorzugte Liganden L" solcher Komplexverbindungen sind z. B. Halogenid-, besonders CI, Br und I, Dien-, besonders Cyclooctadien, Norbornadien-, Olefin-, besonders Ethylen und Cycloocten, Tri- und Tetrahapto-Liganden, besonders 2-Methylallyl-, Indenyl-, Pentamethylcyclopentadienyl- und Cyclopentadienyl-Anion-, Acetato-, Trifluoracetato-, Acetylacetonato- und Hexafluoroacetylacetonato-, PF₃-. sowie Carbonyl- und Hydrido-Liganden.

Bevorzugte koordinierende Lösungsmittel S sind Amine, besonders Triethylamin und Pyridin, Alkohole, besonders Methanol, Ethern, besonders THF und Dioxan, Carbonsäureamide, besonders DMF und Aromaten, besonders Benzol und Cumol.

Bevorzugte nichtkoordinierende Anionen A sind p-Toluolsulfonat, Methylsulfonat, Trifluormethylsulfonat, BF₄; ClO₄, PF₆, und BAr₄.

Die Katalysatoren können unmittelbar vor oder während Reaktion aus dem Metallprecursor und dem Liganden in situ oder in einem inerten Lösungsmittel erzeugt werden. Sie können aber auch vorab hergestellt und gegebenenfalls in isolierter Form eingesetzt werden.
Der Katalysator wird üblicherweise in Mengen von 0.001 bis 10 mol-%, bevorzugt 0.01 bis 5 mol-%, insbesondere 0.1 bis 1 mol% bezogen auf die Carbonylkomponente der Formel (I) eingesetzt.
Die Menge an Ligand liegt vorzugsweise im Bereich von 0.01 bis 50 mol-Äquivalente, bevorzugt 0.5 bis 5 mol-Äquivalente, insbesondere 1 bis 2 mol-Äquivalente, bezogen auf die Menge an eingesetzten Metallprecursor.

Der Katalysator kann auch in trägergebundener Form eingesetzt werden, wobei Alumosislicate, Siliciumdioxide wie Kieselgele oder Kieselgure, Aktivkohle, Aluminiumoxyde oder Zeolithe als Trägermaterialien zu nennen sind.

Als Wasserstoffdonatoren werden primäre und sekundäre Alkohole, hydroaromatische und heterocyclische Verbindungen, Terpene, N-Benzylanilin, Hydrazin, Trialkylsilane, Trialkylzinnhydride, Carbonsäuren und Phosphinigsäuren und deren Estern, Amiden und Ammonium Salzen und deren Gemische angewandt. Bei den primären und sekundären Alkoholen, die als Wasserstoffdonatoren verwendet werden können, handelt es sich um linearen und verzweigten aliphatischen, aliphatisch-aromatischen, cyclischen Alkoholen oder Phenolen, die jeweils 1 bis 20, bevorzugt 1 bis 6, besonders bevorzugt 2 bis 4 Kohlenstoffatome enthalten. Ferner können auch Kohlenhydrate und Polyvinylalkohole angewandt werden. Beispiele für Alkoholen sind Ethanol, Ethylenglykol, Propanol, Isopropanol, Butanol, Isobutanol, Hexandiol-1,6, Cyclopentanol, Cyclohexanol, Phenol, Hydrochinon, Benzylakohol, Benzhydrol und Glucose. Besonders bevorzugt ist Isopropanol.
Bei den hydroaromatische und heterocyclische Verbindungen, die als Wasserstoffdonatoren erfindugsgemäß verwendet werden können, handelt es sich um partiell hydrierten Aromaten und Heteroaromaten, sowie gesättigen, heterocyclischen Verbindungen. Beispiele für hydroaromatische und heterocyclische Verbindungen sind Cyclohexen, Cyclohexadien, Indan, Tetralin, Dihydrofuran, Pyrrolidin, Piperidin, Dioxan, Indolin und Tetrahydrochinolin.
Bei den Carbonsäuren, welche als Wasserstoffdonatoren erfindugsgemäß verwendet werden können, handelt es sich um linearen oder verzweigten Alkancarbonsäuren, die jeweils 1 bis 20, bevorzugt 1 bis 6 Kohlenstoffatome enthalten und gegebenenfalls eine oder mehrere Gruppe aufweisen, die aus der Gruppe Alkenyl, Aryl, CO-Alkyl-(C₁-C₁₀), CO-Aryl-(C₆-C₁₀), COOH, COO-Alkyl-(C₁-C₁₀) und OH ausgewählt werden können. Beispiele für Carbonsäuren sind Ameisensäure, Ascorbinsäure, Milchsäure und Phenylbrenztraubensäure. Ester im Sinne der vorliegenden Erfindung wird aus eine Carbonsäure und (C₁-C₁₀)-Alkohol gebildet.

In einer bevorzugten Ausführung werden Carbonsäuren als Amin- oder AmmoniumSalze eingesetzt, wobei Amin und Ammonium-Kation für Ammoniak oder einen aromatischen oder nicht-aromatischen primären, sekundären oder tertiären Amin und Ammonium-Kation sowie quartären Ammonium-Kation, die jeweils 1 bis 20, bevorzugt 1 bis 6 Kohlenstoffatome enthalten, stehen. Beispiele für Amine sind Trimethylamin, Triethylamin, Diisopropylethylamin und Pyridin. Besonders bevorzugt sind Ammoniumformiat, Triethylammoniumformiat und Ameisensäure-Triethylamin Mischung

Das erfindungsgemäße Verfahren zur Hydrido-transfer reduktive Aminierung von Carbonylverbindungen wird besonders bevorzugt mit Isopraponol, Ammoniumformiat, Triethylammoniumformiat oder Ameisensäure-Triethylamin Mischung als Wasserstoffdonor durchgeführt. Auch Wassersoff als Coreduktant kann eingesetzt werden.

Für das erfindungsgemäße Verfahren kann es Vorteilhaft sein, in Gegenwart von Additiven zu Arbeiten. Additive sind Basen wie Natronlauge, Kalilauge, tertiäre Amine, Protonenschwamm, Cäsiumcarbonat, Acetat, Soda, Salze wie die Halogenide der Alkalimetalle oder Halogenide von Ammoniumsalzen, Phasen-Transfer Katalysatoren, Tenside, Cyclodextrine, die von 0-100 mol% bezogen auf die eingesetzte Carbonylkomponente (I) angewandt werden.
Die Hydridotransfer-Aminierung wird vorzugsweise in Lösung durchgeführt. Als Lösungsmittel eignen sich z. B. Alkohole, insbesondere C₁-C₆-Alkanole, insbesondere bevorzugt Methanol, Ethanol, Propanol, Isopropanol, Butanol oder aber auch Wasser und Gemische derselben. Bei schlecht löslichen Substraten sind auch Lösungsmittelgemische von Alkoholen und halogenierten Kohlenwasserstoffen wie Chloroform und/oder Ethern, insbesondere cyclischen Ethern wie THF, und/oder Kohlenwasserstoffen wie Hexan oder Toluol geeignet. Wird Katalysator in einem dafür geeigneten Lösungsmittel generiert, so wird Katalysator-Lösung zur Reaktionsmischung zugefügt.

Das erfindungsgemäße Verfahren wird in der Regel bei einer Temperatur von -78 bis150 °C, bevorzugt bei -20 bis 110 °C, insbesondere bei 0 bis 80 °C durchgeführt.

Die Reaktionsdauer liegt je nach Substrat, Amin und Temperatur im Bereich von 30 min bis 24 Stunden.

Die nachstehenden Beispiele dienen der Erläuterung der Erfindung, ohne diese darauf zu beschränken.

### Beispiel 1

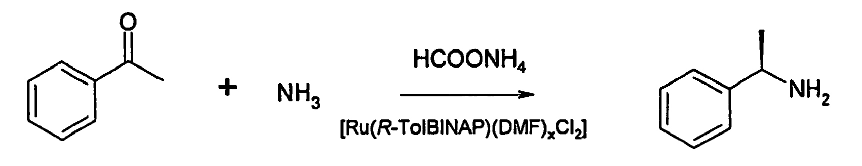

In einem Druckbehälter wurde 240 mg (2 mmol) Acetophenon mit 0.63 g Ammoniumformiat, 40 mg (0.04 mmol) [Ru(*R*-TolBINAP)(DMF)ₓCl₂] und 4 ml 20 % Ammoniaklösung versetzt und bei 100 °C 16 Stunden gerührt. Nach dem Erkalten wurde das Gemisch gas-chromatographisch untersucht. Unter diesen Bedingungen wurde das Keton zu 4 % zu 1-Phenylethanol und zu 96 % zum (*R*)-1-Phenylethylamin mit eine optische Reinheit von 93 % ee umgesetzt.

### Beispiele 2-10

Diese Beispiele werden analog zu Beispiel 1 durchgeführt. Katalysatoren sowie die Reaktionsergebnisse sind in Tabelle 1 angegeben.

**Tabelle 1.**

| | | | | | | | |
|---|---|---|---|---|---|---|---|
| 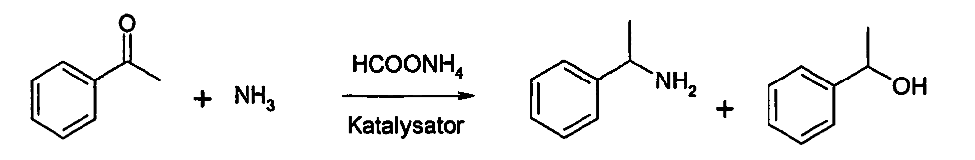 | | | | | | | |

| | Katalysator | T (°C) | Zeit (h) | Edukt | 1-Phenyl ethylamin | ee (%)^{e} | 1-Phenyl ethanol |
|---|---|---|---|---|---|---|---|
| 2 | [Ru(R-ToIBINAP)(R-DAIPEN)Cl₂]^{b} | 80 | 17 | 4 | 96 | 90(R) | |
| 3 | [Ru(S-BINAP)(DMF)ₓCl₂] | 100 | 16 | 0 | 78 | 83(S) | 21 |
| 4 | [Ru(PPh₃)₃Cl₂] | 80 | 7 | 72 | 17 | - | 10 |
| 5 | [Ru(PPh₃)₂(C₅H₅)Cl] | 80 | 7 | 6 | 78 | - | 15 |
| 6 | [Ir(C₈H₁₂)Cl]₂ | 80 | 12 | 15 | 84 | - | 1 |
| 7 | [Ir(C₈H₁₂)Cl]₂ + Bpy^{c} | 80 | 7 | 19 | 81 | - | 0 |
| 8 | [Ir(C₈H₁₄)₂Cl]₂ + S-Norphos^{d} | 80 | 24 | 0 | 63 | 23(R) | 36 |
| 9 | [Ir(C₈H₁₄)₂Cl]₂ + R-TolBINAP | 80 | 24 | 14 | 73 | 0 | 12 |
| 10 | [Rh(C₅Me₅)C₂l]₂ | 80 | 24 | 0 | 98 | - | 1 |

| | | | | | | | |
|---|---|---|---|---|---|---|---|
| ^{a} Bedingungen: 2 mmol Acetophenon, 10 mmol Ammoniumformiat, 0.04 mmol Präkatalysator, 4 ml 20 % Ammoniaklösung in MeOH; ^{b} *R*-DAIPEN = (2R)-(-)-1,1-Bis(4-methoxyphenyl)-3-methyl-1,2-butanediamine; ^{c} Bpy = 2,2'-Bipyridyl;^{d} *S-*Norphos = (2*S*,3*S*)-(+)-2,3-Bis(diphenylphosphino)-bicyclo[2.2.1]hept-5-en; ^{e} GC-Analyse von N-Trifluoracetyl-Derivat auf der chirale Säule CP-Chirasil-Dex CB. | | | | | | | |

### Beispiel 11.

51.5 mg (0.16 mmol) [Ru(COD)(Methallyl)₂] und 122 mg (0.18 mmol) *R*-TolBINAP wurden in 8 ml CH₂Cl₂ gelöst und 48 mkl Tetrafluoroborsäure in Et2O zugeben, anschließend 1ml (0.02 mmol) Katalysator Lösung wurde zu einer Lösunung von 240 mg (2 mmol) Acetophenon und 87 mg Ameisensäure-Trethylamin-Komplex (5:2) in 4 ml 20 % Ammoniaklösung in MeOH gegeben und in einem Druckbehälter bei 60 °C 12 Stunden gerührt. Nach dem Erkalten wurde das Gemisch gas-chromatographisch untersucht. Unter diesen Bedingungen wurde das Keton zu 49 % zum (*R*)-1-Phenylethylamin mit eine optische Reinheit von 90 % ee umgesetzt.

### Beispiele 12-13

Diese Beispiele werden analog zu Beispiel 11 durchgeführt. Katalysatoren sowie die Reaktionsergebnisse sind in Tabelle 2 angegeben.

**Tabelle 2.^{a}**

| | | | | | | | |
|---|---|---|---|---|---|---|---|
| 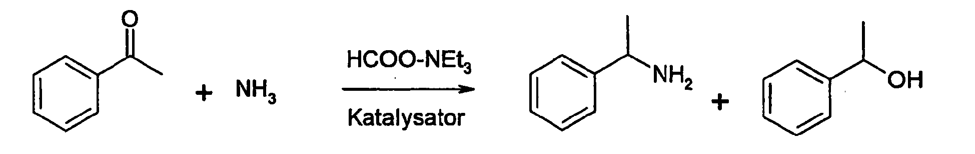 | | | | | | | |

| | Katalysator | T (°C) | Zeit (h) | Edukt | 1-Phenyl ethylamin | ee (%)^{b} | 1-Phenyl ethanol |
|---|---|---|---|---|---|---|---|
| 12 | [Ru(S-BINAP)(DMF)ₓCl₂] | 70 | 38 | 4 | 48 | 80(S) | 47 |
| 13 | [Rh(C₅Me₅)C₂l]₂ | 60 | 12 | 46 | 53 | - | 0 |

| | | | | | | | |
|---|---|---|---|---|---|---|---|
| ^{a} Bedingungen: 2 mmol Acetophenon, 10 mmol Ammoniumformiat, 0.04 mmol Präkatalysator, 4 ml 20 % Ammoniaklösung in MeOH; ^{b} GC-Analyse von N-Trifluoracetyl-Derivat auf der chirale Säule CP-Chirasil-Dex CB. | | | | | | | |

### Beispiel 14.

49.4 mg (0.08 mmol) [Rh(C₅Me₅)Cl₂]₂ werden in 8 ml Methanol vorgelegt, 100 mkl Triethylamin, anschließend 38.2 mg (0.18 mmol) (1*R*,2*R*)-(+)-1,2-Diphenyl-1,2-ethylendiamin zugesetzt und ca. 15 min gerührt. Dann setzte man 1 ml (0.01 mmol) Katalysator-Lösung zu einer Lösung von 240 mg (2 mmol) Acetophenon in 5 ml 20 % Ammoniaklösung in Isopropanol und rührte man 12 Stunden in einem Druckbehälter bei 60 °C. Unter diesen Bedingungen wurde das Keton zu 30 % zum 1-Phenylethylamin mit eine optische Reinheit von 2 % ee umgesetzt

### Beispiel 15.

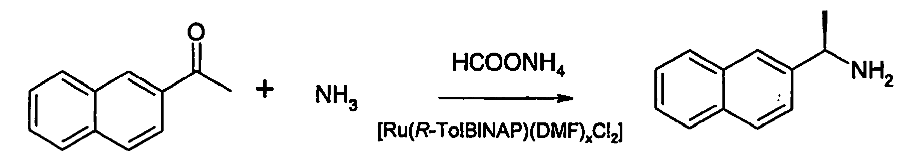

In einem Druckbehälter wurde 85 mg (0.5 mmol) Acetylnaphthalin mit 63 mg Ammoniumformiat, 5 mg (0.005 mmol) [Ru(*R*-TolBINAP)(DMF)ₓCl₂] und 1 ml 20 % Ammoniaklösung versetzt und bei 90 °C 24 Stunden gerührt. Nach dem Erkalten wurde das Gemisch gas-chromatographisch untersucht. Unter diesen Bedingungen wurde das Keton zu 73 % zum (*R*)-1-(Naphthyl-2)ethylamin mit eine optische Reinheit von 99 % ee umgesetzt.

### Beispiele 16-66.

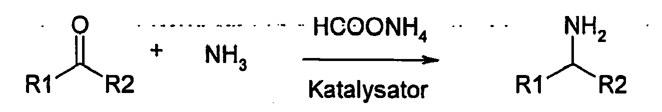

Diese Beispiele werden analog zu Beispiel 15 durchgeführt. Substrate, Katalysatoren sowie die Reaktionsergebnisse sind in Tabelle 3 und 4 angegeben.

**Tabelle 3^{a}**

| | | | | | | |
|---|---|---|---|---|---|---|
| 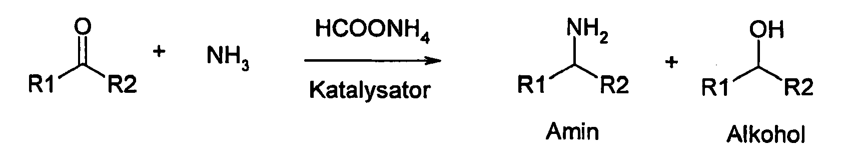 | | | | | | |

| | Substrat | Katalysator ^{b} | Edukt | Amin | ee Amin % | Alkohol |
|---|---|---|---|---|---|---|
| 16 | 2-Acetylnaphthalin | A | 38 | 45 | 90 (S) | 6 |
| 17 | 2-Acetylnaphthalin | C | 38 | 61 | 99 (R) | 0 |
| 18 | 4-Hydroxyacetophenon | A | 0 | 31 | - | 0 |
| 19 | 4-Hydroxyacetophenon | B | 0 | 31 | - | 0 |
| 20 | 4-Hydroxyacetophenon | C | 0 | 28 | - | 0 |
| 21 | 4-Methylacetophenon | A | 0 | 66 | 10 (S) | 0 |
| 22 | 4-Methylacetophenon | B | 0 | 70 | 99 (R) | 0 |
| 23 | 4-Methylacetophenon | C | 0 | 76 | 99 (R) | 0 |
| 24 | 4-Methoxyacetophenon | A | 0 | 84 | 6 (S) | 6 |
| 25 | 4-Methoxyacetophenon | B | 0 | 88 | 99 (R) | 4 |
| 26 | 4-Methoxyacetophenon | C | 0 | 78 | 99 (R) | 16 |
| 27 | 4-Bromacetophenon | A | 0 | 66 | 60 (S) | 2 |
| 28 | 4-Bromacetophenon | B | 0 | 82 | 84 (R) | 0 |
| 29 | 4-Bromacetophenon | C | 0 | 70 | 84 (R) | 0 |
| 30 | 1-Indanon | A | 0.7 | 2.0 | - | 0 |
| 31 | 1-Indanon | B | 0.5 | 4.6 | - | 0 |
| 32 | 1-Indanon | C | 1.9 | 5.7 | - | 0 |
| 33 | 2-Octanon | A | 9 | 20 | - | 22 |
| 34 | 2-Octanon | B | 6 | 28 | - | 8 |
| 35 | 2-Octanon | C | 5 | 24 | - | 22 |

| | | | | | | |
|---|---|---|---|---|---|---|
| Bedingungen: 24 Stunden bei 90 °C, 0.5 mmol Substrat, 1 mmol Ammoniumformiat, 0.005 mmol Katalysator, 1 ml 20 % Ammoniaklösung in MeOH; ^{b} A = [(*S*-BINAP)RuCl₂(DMF)ₓ], B = [(*R*-TolBINAP)RuCl₂(DMF)ₓ], C = [(*R-*TolBINAP)RuCl₂(*R*-DAIPEN)]. | | | | | | |

**Tabelle 4 ^{a}**

| | | | | | |
|---|---|---|---|---|---|
| 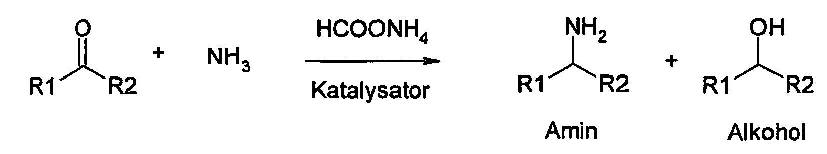 | | | | | |

| | Substrat | Katalysator^{b} | Edukt | Amin | Alkohol |
|---|---|---|---|---|---|
| 36 | 3-Methylacetophenon | A | 1 | 32 | 1 |
| 37 | 3-Methylacetophenon | B | 31 | 14 | 2 |
| 38 | 3-Methylacetophenon | C | 1 | 76 | 1 |
| 39 | 4-Methylacetophenon | A | 0 | 28 | 0 |
| 40 | 4-Methylacetophenon | B | 50 | 23 | 0 |
| 41 | 4-Methylacetophenon | C | 0 | 61 | 0 |
| 42 | 4-Isopropylacetophenon | A | 0 | 35 | 0 |
| 43 | Propiophenon | A | 9 | 36 | 2 |
| 44 | Propiophenon | B | 13 | 2 | 0 |
| 45 | Propiophenon | C | 14 | 73 | 0 |
| 46 | 4-Acetylpyridin | A | 0 | 20 | 28 |
| 47 | 4-Acetylpyridin | B | 15 | 2 | 0 |
| 48 | 4-Acetylpyridin | C | 0 | 33 | 19 |
| 49 | 4-Methoxyacetophenon | A | 0 | 28 | 0 |
| 50 | 4-Methoxyacetophenon | B | 9 | 2 | 0 |
| 51 | 4-Methoxyacetophenon | C | 6 | 44 | 0 |
| 52 | 4-Chloracetophenon | A | 0 | 51 | 0 |
| 53 | 4-Chloracetophenon | C | 4 | 30 | 1 |
| 54 | 4-Bromacetophenon | A | 0 | 37 | 3 |
| 55 | 4-Bromacetophenon | B | 44 | 2 | 2 |
| 56 | 4-Bromacetophenon | C | 7 | 31 | 0 |
| 57 | 1-Acetylnaphthalin | A | 16 | 7 | 5 |
| 58 | 1-Acetylnaphthalin | B | 65 | 6 | 1 |
| 59 | 1-Acetylnaphthalin | C | 30 | 4 | 3 |
| 60 | 2-Acetylnaphthalin | A | 0 | 20 | 0 |
| 61 | 2-Acetylnaphthalin | B | 37 | 10 | 2 |
| 62 | 2-Acetylnaphthalin | C | 3 | 54 | 0 |
| 63 | 2-Octanon | A | 0 | 21 | 3 |
| 64 | 2-Octanon | B | 0 | 17 | 10 |
| 65 | 2-Octanon | C | 0 | 37 | 3 |
| 66 | 2-Methylcyclohexanon | A | 0 | 42^{c} | 0 |

| | | | | | |
|---|---|---|---|---|---|
| ^{a} Bedingungen: 24 Stunden bei 90 °C, 0.5 mmol Substrat, 1 mmol Ammoniumformiat, 0.005 mmol Katalysator, 1 ml 20 % Ammoniaklösung in MeOH; ^{b} A = [(C₅Me₅)RuCl₂], B = [(COD)IrCl]₂, C = [(C₅Me₅) RhCl₂]₂: ^{c} Summe beider Diastereomeren. | | | | | |

### Beispiel 67

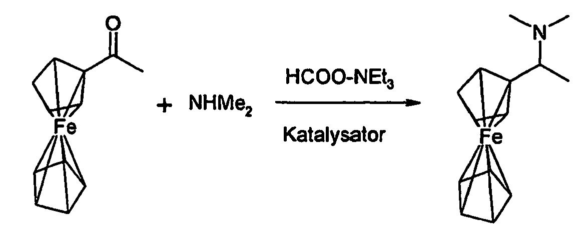

51.5 mg (0.16 mmol) [Ru(COD)(Methallyl)₂] und 122 mg (0.18 mmol) *R*-TolBINAP wurden in 8 ml CH₂Cl₂ gelöst und 48 mkl Tetrafluoroborsäure in Et2O zugeben, anschließend 50 mkl (1 mkmol) Katalysator Lösung wurde zu einer Lösunung von 228 mg (1 mmol) Acetylferrocen und 433 mg Ameisensäure-Trethylamin-Komplex (5:2) in 1 ml 5M Dimethylaminlösung in MeOH gegeben und unter lebhaftem Rühren in einem Druckbehälter bei 80 °C 24 Stunden gerührt. Unter diesen Bedingungen wurde das Keton zu 36 % zum racemischem (1-Dimethylaminoethyl)-ferrocen umgesetzt.

### Beispiele 68-74.

Diese Beispiele werden analog zu Beispiel 67 durchgeführt. Katalysatoren sowie die Reaktionsergebnisse sind in Tabelle 5 angegeben.

**Tabelle 5^{a}**

| | | | | |
|---|---|---|---|---|
| 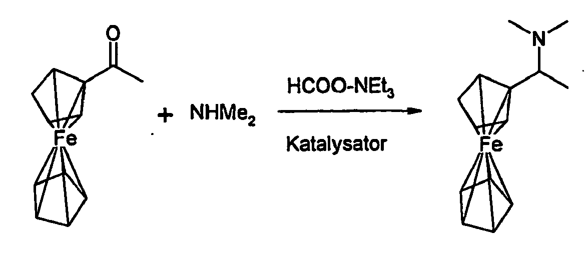 | | | | |

| | Katalysator | Ligand ^{c} | Edukt | Produkt |
|---|---|---|---|---|
| 68 | [Ru(S-BINAP)Cl₂] | | 74 | 13 |
| 69 | [Ru(C₄H₇)₂(C₈H₁₂)] | S-Norphos | 75 | 12 |
| 70 | [Ru(Cymene)Cl₂]₂ | R-DPEA | 78 | 6 |
| 71 | [Ir(C₈H₁₄)₂Cl]₂ | S-Norphos | 75 | 21 |
| 72 | [Ir(C₈H₁₄)₂Cl]₂ | R-DPEA | 70 | 10 |
| 73 | [Ir(C₅Me₅)C₂l]₂ | R-DPEA | 72 | 17 |
| 74 | [Ir(Dmaelnd)(C₈H₁₄)]^{b} | | 67 | 6 |

| | | | | |
|---|---|---|---|---|
| ^{a} Bedingungen: 24 Stunden bei 90 °C, 1 mmol Acetylferrocen, 5 mmol Ameisensäure-Triethylamin, 0.001 mmol Katalysator, 1 ml 5M Dimethylamin-Lösung in Methanol; ^{b} Dmaelnd = 1-(2-Dimethylaminoethyl)indenyl-Anion; ^{c} *S*-Norphos = (2*S*,3*S*)-(+)-2,3-Bis(diphenylphosphino)-bicyclo[2.2.1]hept-5-en, *R*-DPEA= (1R,2R)-(+)-1,2-Diphenyl-1,2-ethylendiamin; | | | | |

### Beispiel 75.

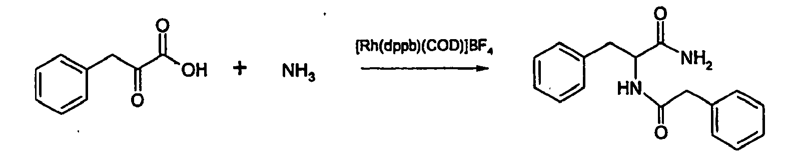

In einem Druckbehälte wurden 3.28 g (20 mmol) Phenylbrenztraubensäure und 72 mg (0.1 mmol) [Rh(dppb)]BF₄ mit 40 ml Methanol versetzt, das bei 10 °C mit Ammoniak gesättigt worden war, und bei 60 °C 10 Stunden lang gerührt. Nach Verdampfen des Lösungsmittels wurde der Rückstand in 15 ml heißem Methanol aufgenommen und die entstandene Lösung abgekühlt, wobei N-Phenylacetylphenylalanin-amid auskristallisierte. Ausbeute 1.42 g (50 %).

### Beispiel 76.

In einem Druckbehälter wurde 106 mg (1 mmol) Benzaldehyd mit 630 mg Ammoniumformiat, 3 mg (0.005 mmol) [Rh(C₅Me₅)Cl₂]₂ und 10 ml 20 % Ammoniaklösung versetzt und bei 60 °C 12 Stunden gerührt. Nach dem Erkalten wurde das Gemisch gas-chromatographisch untersucht. Bei einem Umsatz von 75 % wurde das Verhältnis von 60/15 des Benzylamines zu Dibenzylamin festgestellt.

## Patentansprüche

1. Verfahren zur Herstellung von Aminen durch reduktive Hydridotransfer-Aminierung, wobei Ketone oder Aldehyde mit Ammoniak, primären oder sekundären Aminen in Gegenwart eines Wasserstoffdonors als Reduktionsmittel und eines Katalysators umgesetzt werden, **dadurch gekennzeichnet, dass** Übergangsmetallkomplexkatalysatoren enthaltend mindestens ein Metall aus der Gruppe Fe, Ru, Os, Co, Rh, Ir, Ni, Pd, Pt verwendet werden.

2. Verfahren nach Anspruch 1, **dadurch gekennzeichnet, dass** Katalysatoren enthaltend Rh oder Ru als Übergangsmetallkomplexkatalysator eingesetzt werden.

3. Verfahren nach Anspruch 1 oder 2, **dadurch gekennzeichnet, dass** die eingesetzten Übergangsmetallkatalysatoren mono- oder bidentate Stickstoffdonorliganden, Phosphordonorliganden, Cyclopentadienylliganden, Arenliganden, Olefinliganden, Alkinliganden, Heterocycloalkylliganden Heteroarylliganden Hydridliganden, Alkylliganden, Carbonylliganden oder eine Mischung dieser Liganden enthalten.

4. Verfahren nach einem der Ansprüche 1 bis 3, **dadurch gekennzeichnet, dass** die eingesetzten Übergangsmetallkomplexkatalysatoren mindestens einen mono- oder bidentaten Liganden der Formel (VI) oder (VII) enthalten,
L¹-R¹² (VI)
L¹-Q-L² (VII)
worin,
L¹ und L² unabhängig voneinander für eine koordinierende Gruppe der Formeln (VIII) - (XII) stehen 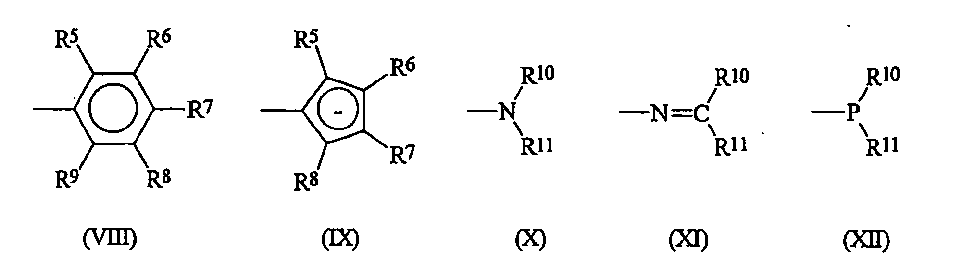
wobei
R⁵ bis R⁹ unabhängig voneinander aus der Gruppe Wasserstoff, (C₁-C₂₄)-Alkyl, (C₂-C₂₄)-Alkenyl, (C₂-C₂₄)-Alkinyl, (C₅-C₁₀)-Aryl, CF₃, CO-Alkyl-(C₁-C₈), CO-Aryl-(C₅-C₁₀), CN, COOH, COOM, COO-Alkyl-(C₁-C₈), COO-Aryl-(C₅-C₁₀), C(=NAlkyl-(C₁-C₈))O-Alkyl-(C₁-C₈), C(=NAryl-(C₅-C₁₀))(O-Alkyl-(C₁-C₈)), C(=NAlkyl-(C₁-C₈))(O-Aryl-(C₅-C₁₀)), C(=NAryl-(C₅-C₁₀))(O-Aryl-(C₅-C₁₀)), CONH₂, CONHAlkyl-(C₁-C₈), CON(Alkyl-(C₁-C₈))₂, CONHAryl-(C₅-C₁₀), CON(Aryl-(C₅-C₁₀))₂, PO(Aryl-(C₅-C₁₀))₂, PO(Alkyl-(C₁-C₄))₂, POH(Alkyl-(C₁-C₆)), POH(Aryl-(C₅-C₁₀)), PO(Alkyl-(C₁-C₄))(O-Alkyl-(C₁-C₆)), PO(Alkyl-(C₁-C₆))(OAryl-(C₅-C₁₀)), PO(Aryl-(C₅-C₁₀))(OAlkyl-(C₁-C₆)), PO₃H₂, PO₃M₂, PO(O-Alkyl-(C₁-C₆))₂, PO(OAryl-(C₅-C₁₀))₂, PO(O-Alkyl-(C₁-C₆))(O-Aryl-(C₅-C₁₀)), SO₃H, SO₃M, SO₃-Alkyl-(C₁-C₄), SO₃-Aryl-(C₅-C₁₀), SO₂-Alkyl-(C₁-C₆), SO₂-Aryl-(C₅-C₁₀), SO-Alkyl-(C₁-C₈), SO-Aryl-(C₅-C₁₀), S-Alkyl-(C₁-C₈), S-Aryl-(C₅-C₁₀), SH, Si(Alkyl-(C₁-C₈))₃, Si(C₁-C₁₀-Alkyl/C₅-C₁₀-Aryl)₃, NO₂, F, CI, Br, I, O-Alkyl-(C₁-C₈), O-Aryl-(C₅-C₁₀), OH, NH₂, NH(Alkyl-(C₁-C₈)), N(Alkyl-(C₁-C₈))₂, NH(Aryl-(C₅-C₁₀)), NHCO-Alkyl-(C₁-C₄), NHCO-Aryl-(C₅-C₁₀), NHCOO-Alkyl-(C₁-C₄), NHCOO-Aryl-(C₅-C₁₀), OCO-Alkyl-(C₁-C₈), OCO-Aryl-(C₅-C₁₀), OPO(Aryl-(C₅-C₁₀))₂, OPO(Alkyl-(C₁-C₄))₂, OPOH(Alkyl-(C₁-C₆)), OPO₃H₂, OPO₃M₂, OPOAlkyl-(C₁-C₄)(O-Alkyl-(C₁-C₆)), OPO(O-Alkyl-(C₁-C₆))₂. OPO(OAryl-(C₅-C₁₀))₂, OSO₃H, OSO₃M, OSO₂-CF₃, OSO₃-Alkyl-(C₁-C₄), OSO₃-Aryl-(C₅-C₁₀), OSO₂-Alkyl-(C₁-C₆), OSO₂-Aryl-(C₅-C₁₀), wobei M ein Kation Li⁺, Na⁺, K⁺, Mg²⁺, Ca²⁺, NH₄⁺ , N(C₁-C₁₀-Alkyl)₄⁺, N(C₁-C₁₀-Alkyl/C₅-C₁₀-Aryl)₄⁺ darstellt, ausgewählt sind, und wobei die Reste
R¹⁰ bis R¹² unabhängig voneinander einen Wasserstoff, (C₁-C₂₄)-Alkyl, (C₂-C₂₄)-Alkenyl, (C₂-C₂₄)-Alkinyl, C₅-C₈ Cycloalkenyl, (C₅-C₁₄)-Aryl, O-Alkyl-(C₁-C₈), O-Aryl-(C₅-C₁₄), O-Alkenyl-(C₂-C₂₄), O-Alkinyl-(C₂-C₂₄), O-Cycloalkenyl-(C₅-C₈), O-Aryl-(C₅-C₁₄), F, NH₂, NH(Alkyl-(C₁-C₈)), NH-Alkenyl-(C₂-C₂₄), NH-Alkinyl-(C₂-C₂₄), NH-Cycloalkenyl-(C₅-C₈), NH-Aryl-(C₅-C₁₄), N(Alkyl-(C₁-C₈))₂, N(Alkenyl-(C₂-C₂₄))₂, N(Alkinyl-(C₂-C₂₄))₂, N(Cycloalkenyl-(C₅-C₈))₂, N(Alkyl-(C₁-C₈))(Aryl-(C₅-C₁₀)), N(Aryl-(C₅-C₁₀))₂, NHCO-Alkyl-(C₁-C₄), NHCO-Alkenyl-(C₂-C₂₄), NHCO-Alkinyl-(C₂-C₂₄), NHCO-Cycloalkenyl-(C₅-C₈), NHCO-Aryl-(C₅-C₁₄), OCO-Alkyl-(C₁-C₄), OCO-Alkenyl-(C₂-C₂₄), OCO-Cycloalkenyl-(C₅-C₈), OCO-Aryl-(C₅-C₁₄), SO₂-Alkyl-(C₁-C₆), SO₂-Aryl-(C₅-C₁₀)-Rest darstellen können und
bei denen alle obengenannten Substituenten jeweils ein- oder mehrfach substituiert sein können, wobei zwei benachbarte Reste gemeinsam für 3 bis 15 Atome aufweisende gesättigte oder ungesättigte carbocyclische oder heterocyclische Gruppen, mit ein bis vier Stickstoff-, Phosphor-, Silicium-, Schwefel- oder Sauerstoffatomen, bilden können und worin
Q eine Brücke, die die Einheit L¹ mit der Einheit L² verbindet, der Formel (XIII) darstellt:
X¹-Z-X² (XIII)
wobei
X¹ und X² unabhängig voneinander eine direkte Bindung oder eine Gruppe -O-, -S-, oder -NR¹³- darstellt, wobei R¹³ einem der für R¹⁰-R¹² definierten Reste sein kann und
Z eine direkte Bindung oder eine Gruppe aus durch Einfach- oder Mehrfachbindungen verknüpften 1-16 Kohlenstoffatome sein kann, wobei ein bis vier Kohlenstoffatome durch Heteroatome ersetzt sein können, wobei Z wie für R⁵-R⁹ angegeben ein oder mehrfach substituiert sein kann oder einen Keto- (=O), Thioketo-(=S) oder Imidsubstituenten (=NR¹⁴) tragen kann, wobei R¹⁴ einer der für R¹⁰-R¹² definierten Reste sein kann und
worin zwei der Einheiten L¹ mit R¹² und L¹ oder L² mit Q miteinander verknüpft sein können, so dass ein 3 bis 15 Atome aufweisendes.gesättigtes oder ungesättigtes carbocyclisches oder ein gesättigtes oder ungesättigtes ein bis vier Stickstoff-, Phosphor-, Silizium-, Schwefel- oder Sauerstoffatome enthaltendes, heterocyclisches Gerüst entsteht.

5. Verfahren nach Anspruch 4, **dadurch gekennzeichnet, dass** Q in Formel (VII) eine aus ein bis vierzehn Kohlenstoffatomen bestehende aliphatischen, olefinische oder acetylenische Brücke darstellt, wobei ein bis vier Kohlenstoffatome gegen Stickstoff- oder Siliciumatome oder ein bis zwei Kohlenstoffatome gegen Sauerstoff oder Schwefelatome ausgetauscht sein können und wobei die einzelnen Brückenglieder der Gruppe unabhängig voneinander Substituenten tragen können wie sie für R⁵-R⁹ definiert sind oder einen Keto- (=O), Thioketo- (=S) oder Imidsubstituenten (=NR¹⁴) tragen können wobei R¹⁴ einem der für R¹⁰-R¹² definierten Reste sein kann und worin zwei der Einheiten L¹, L² und Q miteinander verknüpft sein können, so dass ein 5 bis 9 Atome aufweisendes gesättigtes oder ungesättigtes carbocyclisches oder ein gesättigtes oder ungesättigtes ein bis vier Stickstoff-, Phosphor-, Silicon-, Schwefel- oder Sauerstoffatome enthaltendes, heterocyclisches Gerüst entsteht.

6. Verfahren nach einem der Ansprüche 1 bis 5, **dadurch gekennzeichnet, dass** die eingesetzten Übergangsmetallkomplexkatalysatoren mindestens einen mono-, bi-, tri- oder tetradentaten linearen, verzweigten oder polycyclischen, insbesondere mono- und bicyclischen, Alkin, Olefin, konjugierten oder nicht konjugierten Di- Tri- oder Tetraen mit zwei bis zwölf Kohlenstoffatomen; wobei ein bis vier Kohlenstoffatome gegen Stickstoff- oder Siliciumatome oder ein bis zwei Kohlenstoffatome gegen Sauerstoff- oder Schwefelatome ausgetauscht sein können und wobei die einzelnen Glieder der Gruppe unabhängig voneinander Substituenten tragen können wie sie für R⁵ - R⁹ definiert sind oder einen Keto- (=O), Thioketo- (=S) oder Imidsubstituenten (=NR¹⁴) tragen können, wobei R¹⁴ einem der für R¹⁰-R¹² definierten Reste sein kann.

7. Verfahren nach einem der Ansprüche 1 bis 6, **dadurch gekennzeichnet, dass** die eingesetzten Übergangsmetallkomplexkatalysatoren mindestens einen mono- oder bidentate polycyclischen, insbesondere mono- und bicyclischen gemeinsam für 3 bis 15 Atome aufweisende gesättigte oder ungesättigte heterocyclischen Ligand , mit ein bis vier Sauerstoff , Schwefel- oder Stickstoffatome oder (C₃-C₁₃)-Heteroaromat, mit ein bis vier Sauerstoff-, Schwefel-, Stickstoff- oder ein bis zwei Phosphoratome, wobei die einzelnen Brückenglieder unabhängig voneinander Substituenten tragen können wie sie für R⁵ - R⁹ definiert sind oder einen Keto- (=O), Thioketo- (=S) oder Imidsubstituenten (=NR¹⁴) tragen können, wobei R¹⁴ einem der für R¹⁰-R¹² definierten Reste sein kann.

8. Verfahren nach einem der Ansprüche 1 bis 7, **dadurch gekennzeichnet, dass** als Ligand eine Verbindung ausgewählt aus der Gruppe DAIPEN, BINAP, Dnaelnd, dppb, dcypb, (R,R)-DIPAMP; (R)-Norphos; (R,R)-CHIRAPHOS; (R,R)-DEGUPHOS; (R)-CyGanterPhos; (R,R)-Me-DUPHOS; (R,R)-Et-DUPHOS; (R,R)-Me-BPE; (R,R)-Et-BPE; (R)-Bis(MePheP)benzol; (R)-PROPHOS; (R,R)-SKEWPHOS; (S)-Phos4; (R,S)-Cy-Fc-etdpp; (R,S)-Cy-Fc-etdCyP; (R,S)-Ph-Fc-etdtBuP; (R,S)-JOSIPHOS; (R)-Carboxybutyl-BINAP; (*R*)-BINAP; (R)-TolBINAP ((R)-2,2'-Bis(di-p-tolylphosphino)-1,1'-binapthyl); (R)-MeO-BIPHEP; (R)-p-Tol-MeO-BIPHEP; (S,S)-1,2-(BDPPmethyl)-cyclohexan; (S,S)-DIOP; (S)-MOD-DIOP; (R)-MeAAPHOS; (S,S)-BPPM-H; (S,S)-BPPM; (R,R)-Phenyl-CAPP; (R)-NAPHOS; Ph-β-Glup; Ph-β-Glup-OH; (Phenyl 2,3-bis(O-diphenylphosphino)-β-D-glucopyranosid); DPOE; (*R,R*)-bdpch; (R,R)-CYLOPP-2-Me; (R,R)-CYCLOPP-4-CF3; (R,R)-CYCLOPP-3,5-Cl; (R,R)-CYCLOPP-3,5-CF3; (R,R)-CYCLOPP-3,5-F; CARBOPHOS-3,5-Me2Ph; GLUCOPHOS-Ph-3,5-Me; R-POP-Bz; (R,S)-Phos3; (S)-CyCy-OxoPRONOP; (S)-Cy,Cy-PRONOP; (S)-CyCyisoALANOP; (R)-PROPRAPHOS; PROPRAHOS-Analog (R)-Cyp-PPP; (R)-PN-Ph; (S)-PN-iPr; (S)-PN-iPr-Me; (S)-PN-tBu; (R)-QUINAP; (R,R)-(S,S)-EtTRAP verwendet wird.

9. Verfahren nach einem der Ansprüche 1 bis 8, **dadurch gekennzeichnet, dass** die verwendeten Übergangsmetallkatalysatoren aus mindestens einem Übergangsmetallsalz oder einem Übergangsmetallvorkomplex enthaltend mindestens ein Metall aus der achten Nebengruppe und mindestens einem Liganden der Formeln (VI) oder (VII) hergestellt werden.

10. Verfahren nach einem der Ansprüche 1 bis 9, **dadurch gekennzeichnet, dass** als Wasserstoffdonatoren primäre und sekundäre Alkohole, hydroaromatische und heterocyclische Verbindungen, Terpene, N-Benzylanilin, Hydrazin, Trialkylsilane, Trialkylzinnhydride, Carbonsäuren und Phosphinigsäuren und deren Estern, Amiden und Ammonium Salzen und deren Gemische angewandt werden.

11. Verfahren nach einem der Ansprüche 1 bis 10, **dadurch gekennzeichnet, dass** 1 bis 20 Kohlenstoffatome enthaltende primären und sekundären Alkohole oder Carbonsäuren als Wasserstoffdonatoren verwendet werden.

12. Verfahren nach einem der Ansprüche 1 bis 11, **dadurch gekennzeichnet, dass** Isopropanol, Ammoniumformiat, Triethylammoniumformiat und Ameisensäure-Triethylamin Mischung als Wasserstoffdonoren verwendet werden.

13. Verfahren nach Anspruch einem der Ansprüche 1 bis 12, **dadurch gekennzeichnet, dass** Carbonylverbindungen der Formel (I) 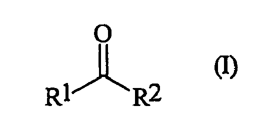 mit Ammoniak, primären oder sekundären Aminen der Formel (II) 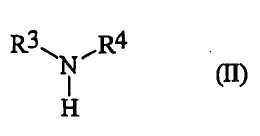 zu Verbindungen der allgemeinen Formel (III) umgesetzt werden, 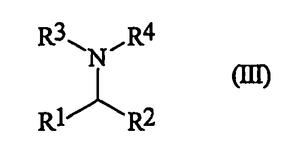 worin die Reste
R¹ bis R⁴ unabhängig voneinander aus der Gruppe von Wasserstoff, (C₁-C₂₄)-Alkyl, (C₂-C₂₄)-Alkenyl, (C₂-C₂₄)-Alkinyl, (C₅-C₁₀)-Aryl, CF₃, CHO, CO-Alkyl-(C₁-C₈), CO-Aryl-(C₅-C₁₀), COO-Alkyl-(C₁-C₈), COO-Aryl-(C₅-C₁₀), C(=NAlkyl-(C₁-C₈))O-Alkyl-(C₁-C₈), C(=NAryl-(C₆-C₁₀))O-Alkyl-(C₁-C₈), C(=NAlkyl-(C₁-C₈))O-Aryl-(C₅-C₁₀), C(=NAryl-(C₅-C₁₀))O-Aryl-(C₅-C₁₀), CONH₂, CONHAlkyl-(C₁-C₈), CON(Alkyl-(C₁-C₈))₂, CONHAryl-(C₅-C₁₀), CON(Aryl-(C₅-C₁₀))₂, PO(Aryl-(C₅-C₁₀))₂, PO(Alkyl-(C₁-C₄))₂, PO(Alkyl-(C₁-C₄))(O-Alkyl-(C₁-C₆)), PO(Alkyl-(C₁-C₆)(OAryl-(C₅-C₁₀)), PO(Aryl-(C₅-C₁₀))(OAlkyl-(C₁-C₆)), PO(O-Alkyl-(C₁-C₆))₂, PO(OAryl-(C₅-C₁₀))₂, PO(O-Alkyl-(C₁-C₆))(O-Aryl-(C₅-C₁₀)), SO₂-OAlkyl-(C₁-C₄), SO₂-OAryl-(C₅-C₁₀), SO₂-Alkyl-(C₁-C₆), SO₂-Aryl-(C₅-C₁₀), SO-Alkyl-(C₁-C₈), SO-Aryl-(C₅-C₁₀) oder Si(Alkyl-(C₁-C₈))₃, Si(C₁-C₁₀-Alkyl/C₅-C₁₀-Aryl)₃, wobei M ein Kation z. B. ein Li⁺, Na⁺, K⁺, Mg²⁺, Ca²⁺, NH₄⁺, N(C₁-C₁₀-Alkyl)₄⁺, N(H/C₁-C₁₀-Alkyl/C₆-C₁₀-Aryl)₄⁺ darstellt, und/oder
R¹ bis R² unabhängig voneinander aus der Gruppe CH(O-Alken-(C₂-C₈)-O), C(O-Alken-(C₂-C₈)-O)-Alkyl-(C₁-C₈), C(O-Alken-(C₂-C₈)-O)-Aryl-(C₅-C₁₀), CH(O-Alkyl-(C₁-C₈))₂, C(O-Alkyl-(C₁-C₈))₂-Alkyl-(C₁-C₈), C(O-Alkyl-(C₁-C₈))₂-Aryl-(C₅-C₁₀), CHO, CN, COOH, COOM, POH(Alkyl-(C₁-C₆)), POH(Aryl-(C₅-C₁₀)), PO₃H₂, PO₃M₂, SO₃H, SO₃M, wobei M ein Kation z. B. ein Li⁺, Na⁺, K⁺, Mg²⁺, Ca²⁺, NH₄⁺, N(C₁-C₁₀-Alkyl)₄⁺, N(H/C₁-C₁₀-Alkyl/C₆-C₁₀-Aryl)₄⁺ darstellt,
und/oder
R³ und R⁴ unabhängig voneinander aus der Gruppe O-Alkyl-(C₁-C₈), O-Aryl-(C₅-C₁₀), Fluor, OH, NH₂, NH(Alkyl-(C₁-C₈)), N(Alkyl-(C₁-C₈))₂, NH(Aryl-(C₅-C₁₀)), NHCO-Alkyl-(C₁-C₄), NHCO-Aryl-(C₅-C₁₀), NHCOO-Alkyl-(C₁-C₈), NHCOO-Aryl-(C₅-C₁₀) darstellt, ausgewählt werden können,
wobei Alkyl für einen nichtverzweigten oder verzweigten aliphatischen oder cyclischen Rest, Alkenyl für einen olefinischen Kohlenwasserstoff, Alkinyl für einen Acetylenkohlenwassersoff und Aryl für einen aromatischen Rest steht, wobei jeweils bis zu 4 Kohlenstoffatome durch Stickstoff-, Phosphor-, Silicium-, Schwefel- oder Sauerstoffatom ausgetauscht sein können und worin sowohl R¹ und R² als auch R³ und R⁴ durch kovalente Bindungen verknüpft sein können, so dass R¹ und R², R³ und R⁴ als auch R¹ und R³ jeweils eine 3 bis 15 Atome aufweisende gesättigte oder ungesättigte carbocyclische oder heterocyclische Einheit, mit ein bis vier Stickstoff-, Phosphor-, Silicium-, Schwefel- oder Sauerstoffatomen, bilden können.

14. Verfahren nach Anspruch 13 **dadurch gekennzeichnet, dass** sowohl die Alkyl-, Alkenyl-, Alkinyl-, Aryl-, Heterocycloalkyl- und Heteroarylgruppen als bevorzugte Substituenten neben Wasserstoff auch (C₁-C₂₀)-Alkyl, (C₂-C₂₀)-Alkenyl, (C₁-C₁₀)-Haloalkyl, (C₃-C₈)-Cycloalkyl, (C₅-C₈)-Cycloalkenyl, (C₂-C₉)-Heterocycloalkyl, (C₁-C₉)-Heterocycloalkenyl, (C₅-C₁₄)-Aryl, (C₂-C₁₃)-Heteroaryl, wobei die Zahl der Heteroatome ausgewählt aus der Gruppe N, O, S, eins bis vier betragen kann, Fluor, Chlor, Brom, Iod, OH, NO₂, CF₃, O-Alkyl-(C₁-C₈), O-Aryl-(C₅-C₁₀), OCO-Alkyl-(C₁-C₈), OCO-Aryl-(C₅-C₁₀), NH₂, NH(Alkyl-(C₁-C₈)), NH(Aryl-(C₅-C₁₀)), N(Alkyl-(C₁-C₈))₂, N(Aryl-(C₅-C₁₀))₂, NHCO-Alkyl-(C₁-C₈), NHCO-Aryl-(C₅-C₁₀), CH(O-Alken-(C₂-C₆)-O), C(O-Alken-(C₂-C₆)-O)-Alkyl-(C₁-C₈), C(O-Alken-(C₂-C₆)-O)-Aryl-(C₅-C₁₀), CH(O-Alkyl-(C₁-C₈))₂, C(O-Alkyl-(C₁-C₈))₂-Alkyl-(C₁-C₈), C(O-Alkyl-(C₁-C₈))₂-Aryl-(C₅-C₁₀), CHO, CO-Alkyl-(C₁-C₈), CO-Aryl-(C₅-C₁₀), CN, COOH, COOM, COO-Alkyl-(C₁-C₈), C(=NAlkyl-(C₁-C₈))O-Alkyl-(C₁-C₈), C(=NAryl-(C₅-C₁₀))O-Alkyl-(C₁-C₈), C(=NAlkyl-(C₁-C₈))O-Aryl-(C₅-C₁₀), C(=NAryl-(C₅-C₁₀))O-Aryl-(C₅-C₁₀), CONH₂, CO-Alkyl-(C₁-C₈), NHCOH, NHCOO-Alkyl-(C₁-C₄), CO-Aryl-(C₅-C₁₀), COO-Aryl-(C₅-C₁₀), CHCH-COOAlkyl-(C₁-C₈), CN, COOH, COOM, CHCHCO₂H, P(Aryl-(C₅-C₁₀))₂, P(Alkyl-(C₁-C₈))₂, PO(Aryl-(C₅-C₁₀))₂, PO(Alkyl-(C₁-C₄))₂, PO₃H₂, PO(Alkyl-(C₁-C₄))(O-Alkyl-(C₁-C₆)), PO(O-Alkyl-(C₁-C₆))₂, OPO(Aryl)₂-(C₅-C₁₀), OPO(Alkyl)₂-(C₁-C₄), OPOH(Alkyl-(C₁-C₆)), OPO₃H₂, OPO₃M₂, OPO(Alkyl-(C₁-C₄))(O-Alkyl-(C₁-C₆)), OPO(Aryl-(C₅-C₁₀))(O-Alkyl-(C₁-C₆)), OPO(O-Alkyl-(C₁-C₆))₂, OPO(OAryl-(C₅-C₁₀))₂, SO₃H, SO₃M, SO₂-O-Alkyl-(C₁-C₄), SO₂-O-Aryl-(C₅-C₁₀), SO₂-Alkyl-(C₁-C₆), SO₂-Aryl-(C₅-C₁₀), SO-Alkyl-(C₁-C₆)), SO-Aryl-(C₅-C₁₀), OSO₃H, OSO₃M, OSO₂-OAlkyl-(C₁-C₄), OSO₂-Alkyl-(C₁-C₆) oder Si(Alkyl-(C₁-C₈))₃, Si(C₁-C₈-Alkyl/C₅-C₁₀-Aryl)₃, wobei M ein Kation aus der Gruppe Li⁺, Na⁺, K⁺, Mg²⁺, Ca²⁺, NH₄⁺, N(C₁-C₁₀-Alkyl)₄⁺, N(H/C₁-C₁₀-Alkyl/C₆-C₁₀-Aryl)₄⁺ darstellt, bedeuten, tragen können.

15. Verfahren nach Anspruch 13, **dadurch gekennzeichnet, dass** die Substituenten der Arylgruppen π-gebundene Metallkomplexe der Formeln (IV) oder (V) sind, 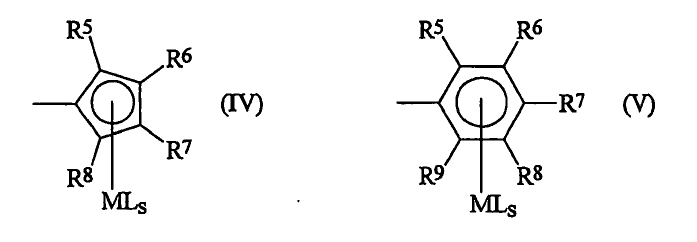 worin
s ganze Zahlen im Bereich von 1 bis 6 und
M Chrom, Molybdän, Wolfram, Mangan, Rhenium, Eisen, Kobalt, Nickel oder ein Element der Lantanidenreihe bedeuten und
R⁵ bis R⁹ gleich oder verschieden sind und einem der für R³-R⁴ definierten Reste entsprechen und worin die Liganden
L₁ bis L_{S} gleich oder verschieden sind und für einen cyclischen Äther mit 5-6 Ringatomen, ein cyclisches Olefin mit 5-8 Ringatomen, Pyridin, CO, PF₃ oder einen Liganden der allgemeinen Formeln (VI) oder (VII) stehen.

16. Verfahren nach einem der Ansprüche 1 bis 15, **dadurch gekennzeichnet, dass** der Übergangsmetallkatalysator in einer Menge zwischen 0.001 bis 10 mol% bezogen auf die Carbonylverbindung eingesetzt wird.

17. Verfahren nach einem der Ansprüche 1 bis 16, **dadurch gekennzeichnet, dass** das Verhältnis zwischen Metallprecursor und Ligand der Formel (VI) oder (VII) bei 1 : 0.01 bis 1 : 50 liegt.

18. Verfahren nach einem der Ansprüche 1 bis 17, **dadurch gekennzeichnet, dass** die Umsetzung bei Temperaturen zwischen 78°C und 150°C durchgeführt wird.

19. Verwendung von Übergangsmetallkomplexkatalysatoren enthaltend mindestens ein Metall aus der Gruppe Fe, Ru, Os, Co, Rh, Ir, Ni, Pd, Pt zur Herstellung von Aminen durch reduktive Hydridotransfer-Aminierung von Ketonen oder Aldehyden mit Ammoniak, primären oder sekundären Aminen in Gegenwart eines Wasserstoffdonors als Reduktionsmittel.

20. Verwendung von Übergangsmetallkomplexkatalysatoren nach Anspruch 19 enthaltend mindestens einen Liganden gemäß der Ansprüche 3 bis 8.

## Claims

1. Process for the production of amines by reductive hydride transfer amination comprising reacting ketones or aldehydes with ammonia, primary or secondary amines in the presence of a hydrogen donor as reducing agent and of a catalyst, **characterized in that** transition metal complex catalysts containing at least one metal from the group Fe, Ru, Os, Co, Rh, Ir, Ni, Pd, Pt are used.

2. Process according to Claim 1, **characterized in that** catalysts containing Rh or Ru are used as transition metal complex catalyst.

3. Process according to Claim 1 or 2, **characterized in that** the transition metal catalysts used contain mono- or bidentate nitrogen donor ligands, phosphorus donor ligands, cyclopentadienyl ligands, arene ligands, olefin ligands, alkyne ligands, heterocycloalkyl ligands, heteroaryl ligands, hydride ligands, alkyl ligands, carbonyl ligands or a mixture thereof.

4. Process according to any one of Claims 1 to 3, **characterized in that** the transition metal complex catalysts used contain at least one mono- or bidentate ligand of formula (VI) or (VII),
L¹-R¹² (VI)
L¹-Q-L² (VII)
where
L¹ and L² independently of one another denote a coordinating group of formulae (VIII)-(XII) 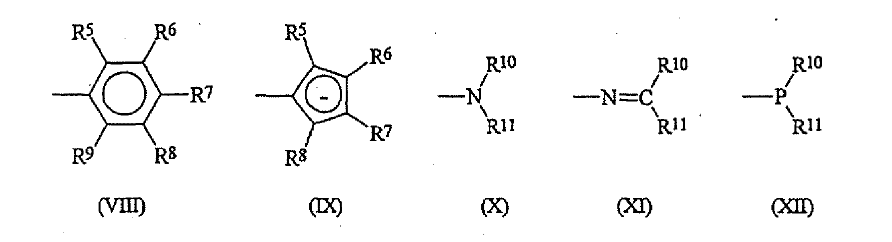
where
R⁵ to R⁹ independently of one another are selected from the group consisting of hydrogen, (C₁-C₂₄)-alkyl, (C₂-C₂₄)-alkenyl, (C₂-C₂₄)-alkynyl, (C₅-C₁₀)-aryl, CF₃, CO-alkyl-(C₁-C₈), CO-aryl-(C₅-C₁₀), CN, COOH, COOM, COO-alkyl-(C₁-C₈), COO-aryl-(C₅-C₁₀), C(=Nalkyl-(C₁-C₈))O-alkyl-(C₁-C₈), C(=Naryl-(C₅-C₁₀))(O-alkyl-(C₁-C₈)), C(=Nalkyl-(C₁-C₈))(O-aryl-(C₅-C₁₀)), C(=Naryl-(C₅-C₁₀))(O-aryl-(C₅-C₁₀)), CONH₂, CONHalkyl-(C₁-C₈), CON(alkyl-(C₁-C₈))₂, CONHaryl-(C₅-C₁₀), CON(aryl-(C₅-C₁₀))₂, PO(aryl-(C₅-C₁₀))₂, PO(alkyl- (C₁-C₄))₂, POH(alkyl-(C₁-C₆)), POH(aryl-(C₅-C₁₀)), PO(alkyl-(C₁-C₄))(O-alkyl-(C₁-C₆)), PO(alkyl-(C₁-C₆))(Oaryl-(C₅-C₁₀)), PO(aryl-(C₅-C₁₀))(Oalkyl-(C₁-C₆)), PO₃H₂, PO₃M₂, PO(O-alkyl-(C₁-C₆))₂, PO(Oaryl-(C₅-C₁₀))₂, PO(O-alkyl-(C₁-C₆))(O-aryl-(C₅-C₁₀)), SO₃H, SO₃M, SO₃-alkyl-(C₁-C₄, SO₃-aryl-(C₅-C₁₀), SO₂-alkyl-(C₁-C₆), SO₂-aryl-(C₅-C₁₀), SO-alkyl-(C₁-C₈) , SO-aryl-(C₅-C₁₀), S-alkyl-(C₁-C₈), S-aryl-(C₅-C₁₀), SH, Si(alkyl-(C₁-C₈))₃, Si(C₁-C₁₀-alkyl/C₅-C₁₀-aryl)₃, NO₂, F, Cl, Br, I, O-alkyl-(C₁-C₈), O-aryl-(C₅-C₁₀), OH, NH₂, NH(alkyl-(C₁-C₈)), N(alkyl-(C₁-C₈))₂, NH(aryl-(C₅₋C₁₀)), NHCO-alkyl-(C₁-C₄), NHCO-aryl-(C₅-C₁₀), NHCOO-alkyl-(C₁-C₄), NHCOO-aryl-(C₅-C₁₀), OCO-alkyl-(C₁-C₈), OCO-aryl-(C₅-C₁₀), OPO(aryl-(C₅-C₁₀))₂, OPO(alkyl-(C₁-C₄))₂, OPOH(alkyl-(C₁-C₆)), OPO₃H₂, OPO₃M₂, OPOalkyl-(C₁-C₄)(O-alkyl-(C₁-C₆)), OPO(O-alkyl-(C₁-C₆))₂, OPO(Oaryl-(C₅-C₁₀))₂, OSO₃H, OSO₃M, OSO₂-CF₃, OSO₃-alkyl-(C₁-C₄), OSO₃-aryl-(C₅-C₁₀), OSO₂-alkyl-(C₁-C₆), OSO₂-aryl-(C₅-C₁₀), where M represents a cation selected from the group consisting of Li⁺, Na⁺, K⁺, Mg²⁺, Ca²⁺, NH₄⁺, N(C₁-C₁₀-alkyl)₄⁺, N(C₁-C₁₀-alkyl/C₅-C₁₀-aryl)₄⁺,
and where the radicals
R¹⁰ to R¹² independently of one another can represent a hydrogen, (C₁-C₂₄)-alkyl, (C₂-C₂₄)-alkenyl, (C₂-C₂₄)-alkynyl, C₅-C₈-cycloalkenyl, (C₅-C₁₄)-aryl, O-alkyl-(C₁-C₈), O-aryl-(C₅-C₁₄), O-alkenyl-(C₂-C₂₄), O-alkynyl-(C₂-C₂₄), O-cyclo-alkenyl-(C₅-C₈), O-aryl-(C₅-C₁₄), F, NH₂, NH(alkyl-(C₁-C₈)), NH-alkenyl-(C₂-C₂₄), NH-alkynyl-(C₂-C₂₄), NH-cycloalkenyl-(C₅-C₈), NH-aryl-(C₅-C₁₄), N(alkyl-(C₁-C₈))₂, N(alkenyl-(C₂-C₂₄))₂, N(alkynyl-(C₂-C₂₄))₂, N(cycloalkenyl-(C₅-C₈))₂, N(alkyl-(C₁-C₈))(aryl-(C₅-C₁₀)), N(aryl-(C₅-C₁₀))₂, NHCO-alkyl-(C₁-C₄), NHCO-alkenyl-(C₂-C₂₄), NHCO-alkynyl-(C₂-C₂₄), NHCO-cycloalkenyl-(C₅-C₈), NHCO-aryl-(C₅-C₁₄), OCO-alkyl-(C₁-C₄), OCO-alkenyl-(C₂-C₂₄), OCO-cycloalkenyl-(C₅-C₈), OCO-aryl-(C₅-C₁₄), SO₂-alkyl-(C₁-C₆), SO₂-aryl-(C₅-C₁₀) radical, and
where all the abovementioned substituents may each be substituted one or more times, in which case two adjacent radicals may together form saturated or unsaturated carbocyclic or heterocyclic groups having 3 to 15 atoms, with one to four nitrogen, phosphorus, silicon, sulphur or oxygen atoms, and where
Q represents a bridge linking the unit L¹ to the unit L², of formula (XIII) :
**X¹-Z-X²** **(XIII)**
where
X¹ and X² independently of one another represent a direct bond or a group -0-, -S- or -NR¹³-, where R¹³ can be one of the radicals defined for R¹⁰ and R¹², and
Z can be a direct bond or a group of 1-16 carbon atoms linked by single or multiple bonds, in which case one to four carbon atoms may be replaced by heteroatoms, in which case Z can be substituted one or more times as stated for R⁵-R⁹ or may carry a keto (=O), thioketo (=S) or imide substituent (=NR¹⁴), in which case R¹⁴ can be one of the radicals defined for R¹⁰-R¹², and
wherein two of the L¹ units can combine with R¹² and L¹ or L² to Q to form a saturated or unsaturated carbocyclic skeleton having 3 to 15 atoms or a saturated or unsaturated heterocyclic skeleton containing one to four nitrogen, phosphorus, silicon, sulphur or oxygen atoms.

5. Process according to Claim 4, **characterized in that** Q in formula (VII) represents an aliphatic, olefinic or acetylenic bridge consisting of one to fourteen carbon atoms, wherein one to four carbon atoms may be replaced by nitrogen or silicon atoms or one to two carbon atoms by oxygen or sulphur atoms and wherein the individual bridging members of the group independently of one another may carry substituents as defined for R⁵ - R⁹ or can carry a keto (=O), thioketo (=S) or imide substituent (=NR¹⁴), in which case R¹⁴ can be one of the radicals defined for R¹⁰-R¹² and where two of the units L¹, L² and Q can be linked together to form a saturated or unsaturated carbocyclic skeleton having 5 to 9 atoms or a saturated or unsaturated heterocyclic skeleton containing one to four nitrogen, phosphorus, silicon, sulphur or oxygen atoms.

6. Process according to any one of Claims 1 to 5, **characterized in that** the transition metal complex catalysts used contain at least one mono-, bi-, tri- or tetradentate linear, branched or polycyclic, especially mono- and bicyclic, alkyne, olefin, conjugated or nonconjugated di-, tri- or tetraene with two to twelve carbon atoms, wherein one to four carbon atoms may be replaced by nitrogen or silicon atoms or one to two carbon atoms by oxygen or sulphur atoms and wherein the individual members of the group independently of one another may carry substituents as defined for R⁵-R⁹ or can carry a keto (=O), thioketo (=S) or imide substituent (=NR¹⁴), in which case R¹⁴ can be one of the radicals defined for R¹⁰-R¹².

7. Process according to any one of Claims 1 to 6, **characterized in that** the transition metal complex catalysts used contain at least one mono- or bidentate polycyclic, especially mono- and bicyclic, saturated or unsaturated heterocyclic ligand having 3 to 15 atoms with one to four oxygen, sulphur or nitrogen atoms, or (C₃-C₁₃) heteroaromatic with one to four oxygen, sulphur, nitrogen or one to two phosphorus atoms, in which case the individual bridging members independently of one another can carry substituents as defined for R⁵-R⁹ or can carry a keto (=0), thioketo (=S) or imide substituent (=NR¹⁴) , where R¹⁴ can be one of the radicals defined for R¹⁰-R¹².

8. Process according to any one of Claims 1 to 7, **characterized in that** the ligand used is a compound selected from the group consisting of DAIPEN, BINAP, Dnaeind, dppb, dcypb, (R,R)-DIPAMP; (R)-Norphos; (R,R)-CHIRAPHOS; (R,R)-DEGUPHOS; (R)-CyGanterPhos; (R,R)-Me-DUPHOS; (R,R)-Et-DUPHOS; (R,R)-Me-BPE; (R,R)-Et-BPE; (R)-bis (MePheP)-benzene; (R)-PROPHOS; (R,R)-SKEWPHOS; (S)-Phos4; (R,S)-Cy-Fc-etdpp; (R,S)-Cy-Fc-etdCyP; (R,S)-Ph-Fc-etdtBuP; (R,S)-JOSIPHOS; (R)-carboxybutyl-BINAP; (*R*)-BINAP; (*R*)-Tol-BINAP ((*R*)-2,2'-bis (di-p-tolylphosphino)-1,1'-binaphthyl); (R)-MeO-BIPHEP; (R)-p-Tol-MeO-BIPHEP; (S,S)-1,2-(BDPPmethyl)cyclohexane; (S,S)-DIOP; (S)-MOD-DIOP; (R)-MeAAPHOS; (S, S) -BPPM-H; (S,S)-BPPM; (R,R)-phenyl-CAPP; (R)-NAPHOS; Ph-β-Glup; Ph-β-Glup-OH; (phenyl 2,3-bis (O-diphenylphosphino)-β-D-glucopyranoside); DPOE; (*R,R*)-bdpch; (R,R)-CYLOPP-2-Me; (R,R)-CYCLOPP-4-CF3; (R,R)-CYCLOPP-3,5-Cl; (R,R)-CYLOPP-3,5-CF3; (R,R)-CYCLOPP-3,5-F; CARBOPHOS-3,5-Me2Ph; GLUCOPHOS-Ph-3,5-Me; *R*-POP-Bz; (R,S)-Phos3; (S)-CyCy-OxoPRONOP; (S)-Cy, Cy-PRONOP; (S)-CyCyisoALANOP; (R)-PROPRAPHOS; PROPRAHOS analogue (*R*)-Cyp-PPP; (R)-PN-Ph; (S)-PN-iPr; (S)-PN-iPr-Me; (S)-PN-tBu; (R)-QUINAP; (R,R)-(S,S)-EtTRAP.

9. Process according to any one of Claims 1 to 8, **characterized in that** the transition metal catalysts used are produced from at least one transition metal salt or from a transition metal pre-complex containing at least one metal from the eighth transition group and at least one ligand of formulae (VI) or (VII).

10. Process according to any one of Claims 1 to 9, **characterized in that** primary and secondary alcohols, hydroaromatic and heterocyclic compounds, terpenes, N-benzylaniline, hydrazine, trialkylsilanes, trialkyltin hydrides, carboxylic acids and phosphinous acids and their esters, amides and ammonium salts and mixtures thereof are used as hydrogen donors.

11. Process according to any one of Claims 1 to 10, **characterized in that** carboxylic acids or primary and secondary alcohols containing 1 to 20 carbon atoms are used as hydrogen donors.

12. Process according to any one of Claims 1 to 11, **characterized in that** isopropanol, ammonium formate, triethylammonium formate and formic acid-triethylamine mixtures are used as hydrogen donors.

13. Process according to any one of Claims 1 to 12, **characterized in that** carbonyl compounds of formula (I) 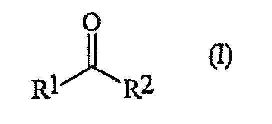 are reacted with ammonia, primary or secondary amines of formula (II) 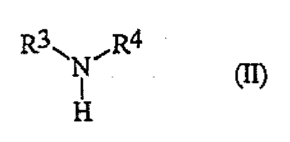 to form compounds of general formula (III) 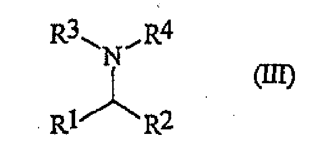 where the radicals
R¹ to R⁴ independently of one another can be selected from the group consisting of hydrogen, (C₁-C₂₄)-alkyl, (C₂-C₂₄)-alkenyl, (C₂-C₂₄)-alkynyl, (C₅-C₁₀)-aryl, CF₃, CHO, CO-alkyl-(C₁-C₈), CO-aryl-(C₅-C₁₀) , COO-alkyl-(C₁-C₈), COO-aryl-(C₅-C₁₀), C(=Nalkyl-(C₁-C₈))O-alkyl-(C₁-C₈), C(=Naryl-(C₆-C₁₀))O-alkyl-(C₁-C₈), C(=Nalkyl-(C₁-C₈))O-aryl-(C₅-C₁₀), C(=Naryl-(C₅-C₁₀))O-aryl-(C₅-C₁₀), CONH₂, CONHalkyl-(C₁-C₈), CON(alkyl-(C₁-C₈))₂, CONHaryl-(C₅-C₁₀), CON (aryl-(C₅-C₁₀))₂, PO(aryl-(C₅-C₁₀))₂, PO (alkyl-(C₁-C₄))₂, PO(alkyl-(C₁-C₄))(O-alkyl(C₁-C₆)), PO(alkyl-(C₁-C₆))(Oaryl-(C₅-C₁₀)), PO(aryl-(C₅-C₁₀))(Oalkyl-(C₁-C₆)), PO(O-alkyl-(C₁-C₆))₂, PO(Oaryl-(C₅-C₁₀))₂, PO(O-alkyl-(C₁-C₆))(O-aryl-(C₅-C₁₀)), SO₂-Oalkyl-(C₁-C₄), SO₂-Oaryl-(C₅-C₁₀), SO₂-alkyl-(C₁-C₆), SO₂-aryl-(C₅-C₁₀), SO-alkyl-(C₁-C₈), SO-aryl-(C₅-C₁₀) or Si(alkyl-(C₁-C₈))₃, Si(C₁-C₁₀-alkyl/C₅-C₁₀-aryl)₃, where M represents a cation, for example an Li⁺, Na⁺, K⁺, Mg²⁺, Ca²⁺, NH₄⁺, N(C₁-C₁₀-alkyl)₄⁺, N(H/C₁-C₁₀-alkyl/C₆-C₁₀-aryl)₄⁺, and/or
R¹ to R² independently of one another can be selected from the group consisting of CH(O-alkene-(C₂-C₈)-O), C(O-alkene-(C₂-C₈)-O)-alkyl-(C₁-C₈), C(O-alkene-(C₂-C₈)-O)-aryl-(C₅-C₁₀), CH(O-alkyl-(C₁-C₈))₂, C(O-alkyl-(C₁-C₈))₂-alkyl-(C₁-C₈), C(O-alkyl-(C₁-C₈))₂-aryl-(C₅-C₁₀), CHO, CN, COOH, COOM, POH(alkyl-(C₁-C₆)), POH(aryl-(C₅-C₁₀)), PO₃H₂, PO₃M₂, SO₃H, SO₃M where M represents a cation, for example an Li⁺, Na⁺, K⁺, Mg²⁺, Ca2⁺, NH₄⁺, N(C₁-C₁₀-alkyl)₄⁺, N(H/C₁-C₁₀-alkyl/C₆-C₁₀-aryl)₄⁺,
and/or
R³ and R⁴ independently of one another can be selected from the group consisting of O-alkyl-(C₁-C₈), O-aryl-(C₅-C₁₀), fluorine, OH, NH₂, NH(alkyl-(C₁-C₈)), N(alkyl-(C₁-C₈))₂, NH(aryl-(C₅-C₁₀)), NHCO-alkyl-(C₁-C₄), NHCO-aryl-(C₅-C₁₀), NHCOO-alkyl-(C₁-C₈), NHCOO-aryl-(C₅-C₁₀),
where alkyl denotes a branched or nonbranched aliphatic or cyclic radical, alkenyl denotes an olefinic hydrocarbon, alkynyl denotes an acetylene hydrocarbon and aryl denotes an aromatic radical, wherein in each case up to 4 carbon atoms can be replaced by nitrogen, phosphorus, silicon, sulphur or oxygen atoms and where not only R¹ and R² but also R³ and R⁴ can be linked by covalent bonds so that R¹ and R², R³ and R⁴ and also R¹ and R³ can in each case form a saturated or unsaturated carbocyclic or heterocyclic unit having 3 to 15 atoms, with one to four nitrogen, phosphorus, silicon, sulphur or oxygen atoms.

14. Process according to Claim 13, **characterized in that** the alkyl, alkenyl, alkynyl, aryl, heterocycloalkyl and heteroaryl groups can carry as preferred substituents, in addition to hydrogen, also (C₁-C₂₀)-alkyl, (C₂-C₂₀)-alkenyl, (C₁-C₁₀)-haloalkyl, (C₃-C₈))-cycloalkyl, (C₅-C₈)-cycloalkenyl, (C₂-C₉)-heterocycloalkyl, (C₁-C₉)-heterocycloalkenyl, (C₅-C₁₄)-aryl, (C₂-C₁₃) - heteroaryl, in which case the number of heteroatoms selected from the group consisting of N, O and S can be one to four, fluorine, chlorine, bromine, iodine, OH, NO₂, CF₃, O-alkyl-(C₁-C₈), O-aryl-(C₅-C₁₀), OCO-alkyl-(C₁-C₈), OCO-aryl-(C₅-C₁₀), NH₂, NH(alkyl-(C₁-C₈)), NH(aryl-(C₅-C₁₀)), N(alkyl-(C₁-C₈))₂, N(aryl-(C₅-C₁₀))₂, NHCO-alkyl-(C₁-C₈), NHCO-aryl-(C₅-C₁₀), CH(O-alkene-(C₂-C₆)-O), C(O-alkene-(C₂-C₆)-O)-alkyl-(C₁-C₈), C(O-alkene-(C₂-C₆)-O)-aryl-(C₅-C₁₀), CH(O-alkyl-(C₁-C₈))₂, C(O-alkyl-(C₁-C₈))₂-alkyl-(C₁-C₈), C(O-alkyl-(C₁-C₈))₂-aryl-(C₅-C₁₀), CHO, CO-alkyl-(C₁-C₈), CO-aryl-(C₅-C₁₀), CN, COOH, COOM, COO-alkyl-(C₁-C₅), C(=Nalkyl-(C₁-C₈))O-alkyl-(C₁-C₈), C (=Naryl-(C₅-C₁₀))O-alkyl-(C₁-C₈), C(=Nalkyl-(C₁-C₈))O-aryl-(C₅-C₁₀), C(=Naryl-(C₅-C₁₀))O-aryl-(C₅-C₁₀), CONH₂, CO-alkyl-(C₁-C₈), NHCOH, NHCOO-alkyl-(C₁-C₄), CO-aryl-(C₅-C₁₀), COO-aryl-(C₅-C₁₀), CHCH-COOalkyl-(C₁-C₈), CN, COOH, COOM, CHCHCO₂H, P(aryl-(C₅-C₁₀))₂, P(alkyl-(C₁-C₈))₂, PO(aryl-(C₅-C₁₀))₂, PO(alkyl-(C₁-C₄))₂, PO₃H₂, PO(alkyl-(C₁-C₄))(O-alkyl-(C₁-C₆)), PO(O-alkyl-(C₁-C₆))₂, OPO(aryl)₂-(C₅-C₁₀), OPO(alkyl)₂-(C₁-C₄), OPOH(alkyl-(C₁-C₆)), OPO₃H₂, OPO₃M₂, OPO(alkyl-(C₁-C₄))(O-alkyl-(C₁-C₆)), OPO(aryl-(C₅-C₁₀))(O-alkyl-(C₁-C₆)), OPO(O-alkyl-(C₁-C₆))₂, OPO(Oaryl-(C₅-C₁₀))₂, SO₃H, SO₃M, SO₂-O-alkyl-(C₁-C₄), SO₂-O-aryl-(C₅-C₁₀), SO₂-alkyl-(C₁-C₆), SO₂-aryl-(C₅-C₁₀), SO-alkyl-(C₁-C₆), SO-aryl-(C₅-C₁₀), OSO₃H, OSO₃M, OSO₂-Oalkyl-(C₁-C₄), OSO₂-alkyl-(C₁-C₆) or Si(alkyl-(C₁-C₈))₃, Si(C₁-C₈-alkyl/C₅-C₁₀-aryl)₃, where M represents a cation from the group Li⁺, Na⁺, K⁺, Mg²⁺, Ca²⁺, NH₄⁺, N (C₁-C₁₀-alkyl)₄⁺, N(H/C₁-C₁₀-alkyl/C₆-C₁₀-aryl)₄⁺.

15. Process according to Claim 13, **characterized in that** the substituents of the aryl groups are π-bound metal complexes of formulae (IV) or (V), 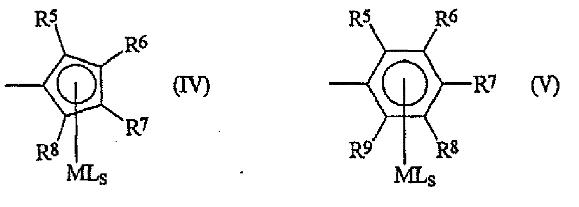 where
s denotes whole numbers in the range from 1 to 6 and
M denotes chromium, molybdenum, tungsten, manganese, rhenium, iron, cobalt, nickel or an element of the lanthanide series, and
R⁵ to R⁹ are the same or different and correspond to one of the radicals defined for R³-R⁴ and
where the ligands
L₁ to Lₛ are the same or different and denote a cyclic ether with 5-6 ring atoms, a cyclic olefin with 5-8 ring atoms, pyridine, CO, PF₃ or a ligand of the general formulae (VI) or (VII).

16. Process according to any one of Claims 1 to 15, **characterized in that** the transition metal catalyst is used in an amount between 0.001 to 10 mol% based on the carbonyl compound.

17. Process according to any one of Claims 1 to 16, **characterized in that** the ratio between metal precursor and ligand of formula (VI) or (VII) is in the range from 1:0.01 to 1:50.

18. Process according to any one of Claims 1 to 17, **characterized in that** the reaction is carried out at temperatures between -78°C and 150°C.

19. Use of transition metal complex catalysts containing at least one metal from the group Fe, Ru, Os, Co, Rh, Ir, Ni, Pd, Pt for production of amines by reductive hydride transfer amination of ketones or aldehydes with ammonia, primary or secondary amines in the presence of a hydrogen donor as reducing agent.

20. Use of transition metal complex catalysts according to Claim 19 containing at least one ligand according to Claims 3 to 8.

## Revendications

1. Procédé de fabrication d'amines par amination réductrice avec transfert d'hydrures, des cétones ou des aldéhydes étant mis en réaction avec de l'ammoniac, des amines primaires ou secondaires, en présence d'un donneur d'hydrogène en tant qu'agent réducteur et d'un catalyseur, **caractérisé en ce que** des catalyseurs à base de complexes de métaux de transition qui contiennent au moins un métal du groupe Fe, Ru, Os, Co, Rh, Ir, Ni, Pd, Pt sont utilisés.

2. Procédé selon la revendication 1, **caractérisé en ce que** des catalyseurs contenant Rh ou Ru sont utilisés en tant que catalyseur à base de complexes de métaux de transition.

3. Procédé selon la revendication 1 ou 2, **caractérisé en ce que** les catalyseurs à base de métaux de transition utilisés contiennent des ligands mono- ou bidentates donneurs d'azote, donneurs de phosphore, cyclopentadiényles, arènes, oléfines, alcynes, hétérocycloalkyles, hétéroaryles, hydrures, alkyles, carbonyles ou un mélange de ces ligands.

4. Procédé selon l'une quelconque des revendications 1 à 3, **caractérisé en ce que** les catalyseurs à base de complexes de métaux de transition utilisés contiennent au moins un ligand mono- ou bidentate de formule (VI) ou (VII),
L¹-R¹² (VI)
L¹-Q-L² (VII)
dans lesquelles
L¹ et L² représentent indépendamment l'un de l'autre un groupe coordinant des formules (VIII) à (XII) 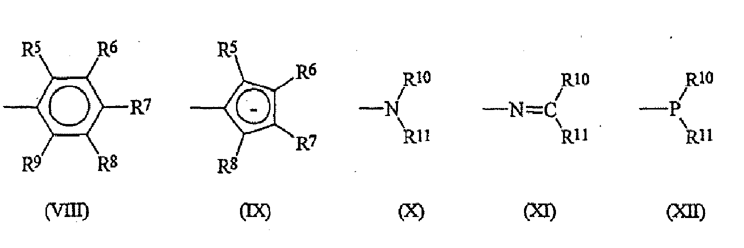
dans lesquelles
R⁵ à R⁹ sont choisis indépendamment les uns des autres dans le groupe constitué par hydrogène, alkyle en C₁-C₂₄, alcényle en C₂-C₂₄, alcynyle en C₂-C₂₄, aryle en C₅-C₁₀, CF₃, CO-alkyl-(C₁-C₈) , CO-aryl-(C₅-C₁₀) CN, COOH, COOM, COO-alkyl-(C₁-C₈), COO-aryl-(C₅-C₁₀), C(=Nalkyl-(C₁-C₈))O-alkyl(C₁-C₈), C(=Naryl-(C₅-C₁₀))(O-alkyl-(C₁-C₈)), C(=Nalkyl-(C₁-C₈))(O-aryl-(C₅-C₁₀)), C(=Naryl-(C₅-C₁₀))(O-aryl-(C₅-C₁₀)), CONH₂, CONHalkyl-(C₁-C₈) , CON(alkyl-(C₁-C₈))₂, CONHaryl-(C₅-C₁₀), CON(aryl-(C₅-C₁₀))₂, PO(aryl-(C₅-C₁₀))₂, PO(alkyl-(C₁-C₄))₂, POH(alkyl-(C₁-C₆)), POH(aryl-(C₅-C₁₀)), PO(alkyl-(C₁-C₄))(O-alkyl-(C₁-C₆)), PO(alkyl-(C₁-C₆))(Oaryl-(C₅-C₁₀)), PO(aryl-(C₅-C₁₀))(Oalkyl-(C₁-C₆)), PO₃H₂, PO₃M₂, PO(O-alkyl-(C₁-C₆))₂, PO(Oaryl-(C₅-C₁₀))₂, PO(O-alkyl-(C₁-C₆))(O-aryl-(C₅-C₁₀)), SO₃H, SO₃M, SO₃-alkyl-(C₁-C₄), SO₃-aryl- (C₅-C₁₀), SO₂-alkyl- (C₁-C₆), SO₂-aryl-(C₅-C₁₀), SO-alkyl-(C₁-C₈), SO-aryl-(C₅-C₁₀), S-alkyl-(C₁-C₈), S-aryl-(C₅-C₁₀), SH, Si (alkyl-(C₁-C₈))₃, Si(C₁-C₁₀-alkyl/C₅-C₁₀-aryl)₃, NO₂, F, Cl, Br, I, O-alkyl-(C₁-C₈), O-aryl-(C₅-C₁₀), OH, NH₂, NH(alkyl-(C₁-C₈)), N(alkyl-(C₁-C₈))₂, NH(aryl-(C₅-C₁₀)), NHCO-alkyl-(C₁-C₄), NHCO-aryl-(C₅-C₁₀), NHCOO-alkyl-(C₁-C₄), NHCOO-aryl-(C₅-C₁₀), OCO-alkyl-(C₁-C₈), OCO-aryl-(C₅-C₁₀), OPO(aryl-(C₅-C₁₀))₂, OPO(alkyl-(C₁-C₄))₂, OPOH(alkyl-(C₁-C₆)), OPO₃H₂, OPO₃M₂, OPOalkyl- (C₁-C₄)(O-alkyl-(C₁-C₆)), OPO(O-alkyl-(C₁-C₆))₂, OPO(Oaryl-(C₅-C₁₀))₂, OSO₃H, OSO₃M, OSO₂-CF₃, OSO₃-alkyl-(C₁-C₄), OSO₃-aryl-(C₅-C₁₀), OSO₂-alkyl-(C₁-C₆), OSO₂-aryl-(C₅-C₁₀), M représentant un cation Li⁺, Na⁺, K⁺, Mg²⁺, Ca²⁺, NH₄⁺, N(C₁-C₁₀-alkyl)₄⁺, N (C₁-C₁₀-alkyl/C₅-C₁₀-aryl)₄⁺,
et dans lesquelles les radicaux
R¹⁰ à R¹² peuvent représenter indépendamment les uns des autres un radical hydrogène, alkyle en C₁-C₂₄, alcényle en C₂-C₂₄, alcynyle en C₂-C₂₄, cycloalcényle en C₅-C₈, aryle en C₅-C₁₄, O-alkyl-(C₁-C₈), O-aryl-(C₅-C₁₄), O-alcényl-(C₂-C₂₄), O-alcynyl- (C₂-C₂₄), O-cycloalcényl-(C₅-C₈), O-aryl-(C₆-C₁₄), F, NH₂, NH(alkyl-(C₁-C₈)), NH-alcényl-(C₂-C₂₄), NH-alcynyl-(C₂-C₂₄), NH-cycloalcényl-(C₅-C₈)),NH-aryl-(C₅-C₁₄), N(alkyl-(C₁-C₈))₂, N(alcényl-(C₂-C₂₄))₂, N(alcynyl-(C₂-C₂₄))₂, N(cycloalcényl-(C₅-C₈))₂, N(alkyl-(C₁-₈))(aryl-(C₅-C₁₀), N(aryl-(C₅-C₁₀))₂, NHCO-alkyl-(C₁-C₄), NHCO-alcényl-(C₂-C₂₄), NHCO-alcynyl- (C₂-C₂₄), NHCO-cycloalcényl-(C₅-C₈), NHCO-aryl-(C₅-C₁₄), OCO-alkyl-(C₁-C₄), OCO-alcényl-(C₂-C₂₄), OCO-cycloalcényl-(C₅-C₈), OCO-aryl-(C₅-C₁₄), SO₂-alkyl-(C₁-C₆), SO₂-aryl-(C₅-C₁₀),
tous les substituants susmentionnés pouvant à chaque fois être substitués une ou plusieurs fois, deux radicaux adjacents pouvant former ensemble des groupes carbocycliques ou hétérocycliques, saturés ou insaturés, comportant 3 à 15 atomes, avec un à quatre atomes d'azote, de phosphore, de silicium, de soufre ou d'oxygène, et dans lesquelles
Q représente un pont qui relie l'unité L¹ avec l'unité L², de formule (XIII) :
X¹-Z-X² (XIII)
dans laquelle
X¹ et X² représentent indépendamment l'un de l'autre une liaison directe ou un groupe -O-, -S- ou -NR¹³-, R¹³ pouvant être un des radicaux définis pour R¹⁰ à R¹² et
Z peut être une liaison directe ou un groupe de 1 à 16 atomes de carbone reliés par des liaisons simples ou multiples, un à quatre atomes de carbone pouvant être remplacés par des hétéroatomes, Z pouvant être substitué une ou plusieurs fois tel qu'indiqué pour R⁵ à R⁹ ou pouvant porter un substituant céto (=O), thiocéto (=S ) ou imide (=NR¹⁴), R¹⁴ pouvant être un des radicaux définis pour R¹⁰ à R¹² et
dans lesquelles deux des unités L¹ avec R¹² et L¹ ou L² avec Q peuvent être reliées l'une à l'autre, de manière à former un squelette carbocyclique saturé ou insaturé comportant 3 à 15 atomes ou un squelette hétérocyclique saturé ou insaturé contenant un à quatre atomes d'azote, de phosphore, de silicium, de soufre ou d'oxygène.

5. Procédé selon la revendication 4, **caractérisé en ce que** Q dans la formule (VII) représente un pont aliphatique, oléfinique ou acétylénique constitué d'un à quatorze atomes de carbone, un à quatre atomes de carbone pouvant être remplacés par des atomes d'azote ou de silicium ou un à deux atomes de carbone pouvant être remplacés par des atomes d'oxygène ou de soufre et les éléments individuels de pont du groupe pouvant indépendamment les uns des autres porter des substituants tels que définis pour R⁵ - R⁹ ou pouvant porter un substituant céto (=O), thiocéto (=S) ou imide (=NR¹⁴) , R¹⁴ pouvant être un des radicaux définis pour R¹⁰ à R¹², et deux des unités L¹, L² et Q pouvant être reliées l'une à l'autre, de manière à former un squelette carbocyclique saturé ou insaturé comportant 5 à 9 atomes ou un squelette hétérocyclique saturé ou insaturé contenant un à quatre atomes d'azote, de phosphore, de silicium, de soufre ou d'oxygène.

6. Procédé selon l'une quelconque des revendications 1 à 5, **caractérisé en ce que** les catalyseurs à base de complexes de métaux de transition utilisés contiennent au moins un alcyne, une oléfine, un di-, tri- ou tétraène conjugué ou non conjugué comportant deux à douze atomes de carbone, mono-, bi-, tri- ou tétradentate linéaire, ramifié ou polycyclique, notamment mono- et bicyclique, un à quatre atomes de carbone pouvant être remplacés par des atomes d'azote ou de silicium ou un à deux atomes de carbone pouvant être remplacés par des atomes d'oxygène ou de soufre et les éléments individuels du groupe pouvant porter indépendamment les uns des autres des substituants tels que définis pour R⁵ à R⁹ ou pouvant porter un substituant céto (=O), thiocéto (=S) ou imide (=NR¹⁴), R¹⁴ pouvant être un des radicaux définis pour R¹⁰ à R¹².

7. Procédé selon l'une quelconque des revendications 1 à 6, **caractérisé en ce que** les catalyseurs à base de complexes de métaux de transition utilisés contiennent au moins un ligand hétérocyclique saturé ou insaturé, mono- ou bidentate, polycyclique, notamment mono- et bicyclique, comportant conjointement 3 à 15 atomes, avec un à quatre atomes d'oxygène, de soufre ou d'azote ou un composé hétéroaromatique en C₃ à C₁₃, avec un à quatre atomes d'oxygène, de soufre, d'azote ou un à deux atomes de phosphore, les éléments individuels de pont pouvant indépendamment les uns des autres porter des substituants tels que définis pour R⁵ à R⁹ ou pouvant porter un substituant céto (=O), thiocéto (=S) ou imide (=NR¹⁴), R¹⁴ pouvant être un des radicaux définis pour R¹⁰ à R¹².

8. Procédé selon l'une quelconque des revendications 1 à 7, **caractérisé en ce qu'**un composé choisi dans le groupe constitué par DAIPEN, BINAP, Dnaelnd, dppb, dcypb, (R,R)-DIPAMP ; (R)-Norphos ; (R,R)-CHIRAPHOS ; (R,R)-DEGUPHOS ; (R)-CyGanterPhos ; (R,R)-Me-DUPHOS ; (R,R)-Et-DUPHOS ; (R,R)-Me-BPE ; (R,R)-Et-BPE ; (R)-bis(MePheP)benzène ; (R)-PROPHOS ; (R,R)-SKEWPHOS ; (S)-Phos4 ; (R,S)-Cy-Fc-etdpp ; (R,S)-Cy-Fc-etdCyP ; (R,S)-Ph-Fc-etdtBuP ; (R,S)-JOSIPHOS ; (R)-carboxybutyl-BINAP; (R)-BINAP ; (R)-Tol-BINAP ((R)-2,2'-bis(di-p-tolylphosphino)-1,1'-binaphtyl) ; (R)-MeO-BIPHEP ; (R)-p-Tol-MeO-BIPHEP ; (S,S)-1,2-(BDPPméthyl)-cyclohexane ; (S,S)-DIOP ; (S)-MOD-DIOP ; (R)-MeAAPHOS ; (S,S)-BPPM-H ; (S,S)-BPPM ; (R,R)-phényl-CAPP ; (R)-NAPHOS ; Ph-β-Glup ; Ph-β-Glup-OH ; (phényle 2,3-bis(O-diphénylphosphino)-β-D-glucopyranoside); DPOE ; (R,R)-bdpch ; (R,R)-CYLOPP-2-Me ; (R,R)-CYCLOPP-4-CF3 ; (R,R)-CYCLOPP-3,5-Cl ; (R,R)-CYCLOPP-3,5-CF3; (R,R)-CYCLOPP-3,5-F ; CARBOPHOS-3,5-Me2Ph ; GLUCOPHOS-Ph-3,5-Me ; R-POP-Bz ; (R,S)-Phos3 ; (S)-CyCy-OxoPRONOP ; (S)-Cy,Cy-PRONOP ; (S)-CyCyisoALANOP ; (R)-PROPRAPHOS ; analogue de PROPRAHOS (R)-Cyp-PPP ; (R)-PN-Ph ; (S)-PN-iPr ; (S)-PN-iPr-Me ; (S)-PN-tBu (R)-QUINAP ; (R,R)-(S,S)-EtTRAP est utilisé en tant que ligand.

9. Procédé selon l'une quelconque des revendications 1 à 8, **caractérisé en ce que** les catalyseurs à base de métaux de transition utilisés sont fabriqués à partir d'au moins un sel de métal de transition ou un précomplexe de métal de transition contenant au moins un métal du huitième sous-groupe et au moins un ligand de formule (VI) ou (VII).

10. Procédé selon l'une quelconque des revendications 1 à 9, **caractérisé en ce que** des alcools primaires et secondaires, des composés hydroaromatiques et hétérocycliques, des terpènes, de la N-benzylaniline, de l'hydrazine, des trialkylsilanes, des hydrures de trialkylétain, des acides carboxyliques et des acides phosphiniques et leurs esters, amides et sels d'ammonium et leurs mélanges sont utilisés en tant que donneurs d'hydrogène.

11. Procédé selon l'une quelconque des revendications 1 à 10, **caractérisé en ce que** des acides carboxyliques ou des alcools primaires et secondaires contenant 1 à 20 atomes de carbone sont utilisés en tant que donneurs d'hydrogène.

12. Procédé selon l'une quelconque des revendications 1 à 11, **caractérisé en ce que** de l'isopropanol, du formiate d'ammonium, du formiate de triéthylammonium et un mélange acide formique-triéthylamine sont utilisés en tant que donneurs d'hydrogène.

13. Procédé selon l'une quelconque des revendications 1 à 12, **caractérisé en ce que** des composés carbonyles de formule (I) 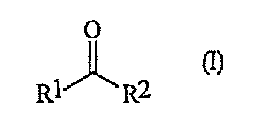 sont mis en réaction avec de l'ammoniac, des amines primaires ou secondaires de formule (II) 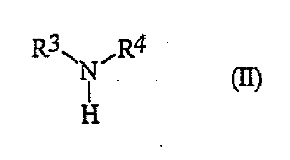 pour former des composés de formule générale (III), 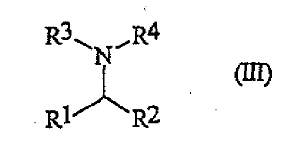 dans laquelle les radicaux
R¹ à R⁴ peuvent être choisis indépendamment les uns des autres dans le groupe constitué par hydrogène, alkyle en C₁-C₂₄, alcényle en C₂-C₂₄, alcynyle en C₂-C₂₄, aryle en C₅-C₁₀, CF₃, CHO, CO-alkyl-(C₁-C₈), CO-aryl-(C₅-C₁₀), COO-alkyl-(C₁-C₈), COO-aryl-(C₅-C₁₀), C(=Nalkyl-(C₁-C₈))O-alkyl-(C₁-C₈), C(=Naryl-(C₆-C₁₀))O-alkyl-(C₁-C₈), C(=Nalkyl-(C₁-C₈))O-aryl-(C₅-C₁₀), C(=Naryl-(C₅-C₁₀))O-aryl-(C₅-C₁₀), CONH₂, CONHalkyl-(C₁-C₈), CON(alkyl-(C₁-C₈))₂, CONHaryl-(C₅-C₁₀), CON(aryl-(C₅-C₁₀))₂, PO(aryl-(C₅-C₁₀))₂, PO(alkyl-(C₁-C₄)₂, PO(alkyl-(C₁-C₄))(O-alkyl-(C₁-C₆)), PO(alkyl-(C₁-C₆))(Oaryl-(C₅-C₁₀)), PO(aryl-(C₅-C₁₀))(Oalkyl-(C₁-C₆)), PO(O-alkyl-(C₁-C₆))₂, PO(Oaryl-(C₅-C₁₀))₂, PO(O-alkyl-(C₁-C₆))(O-aryl-(C₅-C₁₀)), SO₂-Oalkyl-(C₁-C₄), SO₂-Oaryl-(C₅-C₁₀), SO₂-alkyl-(C₁-C₆), SO₂-aryl-(C₅-C₁₀), SO-alkyl-(C₁-C₈), SO-aryl-(C₅-C₁₀) ou Si(alkyl-(C₁-C₈)₃, Si(C₁-C₁₀-alkyl/C₅-C₁₀-aryl)₃, M représentant un cation, p. ex. un Li⁺, Na⁺, K⁺, 2⁺ Ca²⁺, NH₄⁺, N(C₁-C₁₀-alkyl)₄⁺, (H/C₁-C₁₀-alkyl/C₆-C₁₀-aryl)₄⁺ et/ou
R¹ à R² peuvent être choisis indépendamment l'un de l'autre dans le groupe constitué par CH(O-alcène-(C₂-C₈)-O), C(O-alcène-(C₂-C₈)-O)-alkyl-(C₁-C₈), C(O-alcène-(C₂-C₈)-O)-aryl-(C₅-C₁₀), CH(O-alkyl-(C₁-C₈))₂, C(O-alkyl-(C₁-C₈))₂-alkyl-(C₁-C₈), C(O-alkyl-(C₁-C₈))₂-aryl-(C₅-C₁₀), CHO, CN, COOH, COOM, POH(alkyl-(C₁-C₆)), POH(aryl-(C₅-C₁₀)), PO₃H₂, PO₃M₂, SO₃H, SO₃M, M représentant un cation, p. ex. un Li⁺, Na⁺, K⁺, Mg²⁺, Ca²⁺, NH₄⁺, N(C₁-C₁₀-alkyl)₄⁺, (H/C₁-C₁₀-alkyl/C₆-C₁₀-aryl)₄⁺ et/ou
R³ et R⁴ peuvent être choisis indépendamment l'un de l'autre dans le groupe constitué par O-alkyl-(C₁-C8), O-aryl-(C₅-C₁₀), fluor, OH, NH₂, NH(alkyl-(C₁-C₈)), N(alkyl-(C₁-C₈))₂, NH(aryl-(C₅-C₁₀)), NHCO-alkyl-(C₁-C₄)(NHCO-aryl-(C₅-C₁₀), NHCOO-alkyl-(C₁-C₈) NHCOO-aryl-(C₅-C₁₀),
alkyle représentant un radical non ramifié ou ramifié aliphatique ou cyclique, alcényle représentant un hydrocarbure oléfinique, alcynyle représentant un hydrocarbure acétylénique et aryle représentant un radical aromatique, jusqu'à 4 atomes de carbone pouvant à chaque fois être remplacés par un atome d'azote, de phosphore, de silicium, de soufre ou d'oxygène et aussi bien R¹ et R² que R³ et R⁴ pouvant être reliés par des liaisons covalentes, de manière à ce que R¹ et R², R³ et R⁴, ainsi que R¹ et R³ puissent former à chaque fois une unité carbocyclique ou hétérocyclique saturée ou insaturée comportant 3 à 15 atomes, avec un à quatre atomes d'azote, de phosphore, de silicium, de soufre ou d'oxygène.

14. Procédé selon la revendication 13, **caractérisé en ce que** les groupes alkyles, alcényles, alcynyles, aryles, hétérocycloalkyles et hétéroaryles peuvent porter, en tant que substituants préférés, outre l'hydrogène, également alkyle en C₁-C₂₀, alcényle en C₂-C₂₀, halogénoalkyle en C₁-C₁₀, cycloalkyle en C₃-C₈, cycloalcényle en C₅-C₈, hétérocycloalkyle en C₂-C₉, hétérocycloalcényle en C₁-C₉, aryle en C₅-C₁₄, hétéroaryle en C₂-C₁₃, le nombre d'hétéroatomes choisis dans le groupe constitué par N, 0, S pouvant être de un à quatre, fluor, chlore, brome, iode, OH, NO₂, CF₃, O-alkyl-(C₁-C₈), O-aryl-(C₅-C₁₀), OCO-alkyl-(C₁-C₈), OCO-aryl-(C₅-C₁₀), NH₂, NH(alkyl-(C₁-C₈)), NH(aryl-(C₅-C₁₀)), N(alkyl-(C₁-C₈))₂, N(aryl-(C₅-C₁₀))₂, NHCO-alkyl-(C₁-C₈), NHCO-aryl-(C₅-C₁₀), CH(O-alcène-(C₂-C₆)-O), C(O-alcène-(C₂-C₆-O)-alkyl-(C₁-C₈), C(O-alcène-(C₂-C₆)-O)-aryl-(C₅-C₁₀), CH(O-alkyl-(C₁-C₈))₂, C(O-alkyl-(C₁-C₈))₂-alkyl-(C₁-C₈), C(O-alkyl-(C₁-C₈))₂-aryl-(C₅-C₁₀), CHO, CO-alkyl-(C₁-C₈), CO-aryl-(C₅-C₁₀), CN, COOH, COOM, COO-alkyl-(C₁-C₈), C(=Nalkyl-(C₁-C₈))O-alkyl-(C₁-C₈), C(=Naryl-(C₅-C₁₀))O-alkyl-(C₁-C₈), C(=Nalkyl-(C₁-C₈))O-aryl-(C₅-C₁₀), C(=Naryl-(C₅-C₁₀)O-aryl-(C₅-C₁₀), CONH₂, CO-alkyl-(C₁-C₈), NHCOH, NHCOO-alkyl-(C₁-C₄), CO-aryl-(C₅-C₁₀), COO-aryl-(C₅-C₁₀), CHCH-COOalkyl-(C₁-C₈), CN, COOH, COOM, CHCHCO₂H, P(aryl-(C₅-C₁₀))₂, P(alkyl-(C₁-C₈))₂, PO(aryl-(C₅-C₁₀))₂, PO(alkyl-(C₁-C₄)), PO₃H₂, PO(alkyl-(C₁-C₄)) (O-alkyl-(C₁-C₆)), PO(O-alkyl-(C₁-C₆))₂, OPO(aryl)₂-(C₅-C₁₀), OPO(alkyl)₂-(C₁-C₄), OPOH(alkyl-(C₁-C₆)), OPO₃H₂, OPO₃M₂, OPO(alkyl-(C₁-C₄))(O-alkyl-(C₁-C₆)), OPO(aryl-(C₅-C₁₀)) (O-alkyl-(C₁-C₆)), OPO(O-alkyl-(C₁-C₆))₂, OPO(Oaryl-(C₅-C₁₀))₂, SO₃H, SO₃M, SO₂-O-alkyl-(C₁-C₄), SO₂-O-aryl-(C₅-C₁₀), SO₂-alkyl-(C₁-C₆), SO₂-aryl-(C₆-C₁₀), SO-alkyl-(C₁-C₆), SO-aryl-(C₅-C₁₀), OSO₃H, OSO₃M, OSO₂-Oalkyl-(C₁-C₄), OSO₂-alkyl-(C₁-C₆) ou Si(alkyl-(C₁-C₈))₃, Si(C₁-C₈-alkyl/C₅-C₁₀-aryl)₃, M représentant un cation du groupe constitué par Li⁺, Na⁺, K⁺, Mg²⁺, Ca²⁺, NH₄⁺, N(C₁-C₁₀-alkyl)₄⁺, N(H/C₁-C₁₀-alkyl/C₆-C₁₀-aryl)₄⁺.

15. Procédé selon la revendication 13, **caractérisé en ce que** les substituants des groupes aryles sont des complexes métalliques à liaison Π de formule (IV) ou (V) , 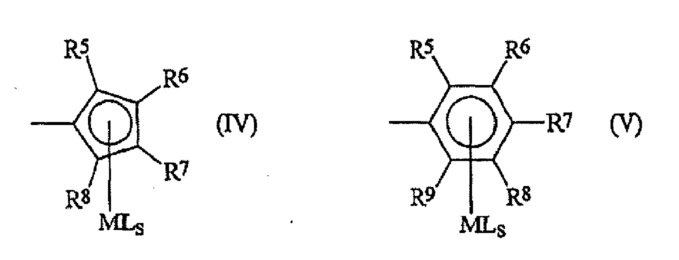 dans lesquelles
s représente des nombres entiers dans la plage allant de 1 à 6 et
M représente le chrome, le molybdène, le tungstène, le manganèse, le rhénium, le fer, le cobalt, le nickel ou un élément de la série des lanthanides et
R⁵ à R⁹ sont identiques ou différents et représentent un des radicaux définis pour R³ à R⁴ et dans lesquelles les ligands
L₁ à L_{S} sont identiques ou différents et représentent un éther cyclique ayant 5 à 6 atomes cycliques, une oléfine cyclique ayant 5 à 8 atomes cycliques, la pyridine, CO, PF₃ ou un ligand de formule générale (VI) ou (VII).

16. Procédé selon l'une quelconque des revendications 1 à 15, **caractérisé en ce que** le catalyseur à base de métaux de transition est utilisé en une quantité comprise entre 0,001 et 10 % en moles par rapport au composé carbonyle.

17. Procédé selon l'une quelconque des revendications 1 à 16, **caractérisé en ce que** le rapport entre le précurseur métallique et le ligand de formule (VI) ou (VII) est de 1:0,01 à 1:50.

18. Procédé selon l'une quelconque des revendications 1 à 17, **caractérisé en ce que** la réaction est réalisée à des températures comprises entre -78 °C et 150 °C.

19. Utilisation de catalyseurs à base de complexes de métaux de transition contenant au moins un métal du groupe constitué par Fe, Ru, Os, Co, Rh, Ir, Ni, Pd, Pt pour la fabrication d'amines par amination réductrice avec transfert d'hydrures de cétones ou d'aldéhydes avec de l'ammoniac, des amines primaires ou secondaires, en présence d'un donneur d'hydrogène en tant qu'agent réducteur.

20. Utilisation de catalyseurs à base de complexes de métaux de transition selon la revendication 19, contenant au moins un ligand selon les revendications 3 à 8.
